# EUROPEAN PATENT SPECIFICATION

(11) **EP 0 178 911 B1**
(45) Date of publication and mention of the grant of the patent: **22.01.1992**
(21) Application number: 85307427.6
(22) Date of filing: 15.10.1985
(51) Int. Cl.: A61K 31/43, A61K 31/40

(54) **Composition containing a penem or carbapenem antibiotic**
Zusammensetzung mit Gehalt an einem Penem- oder Carbapenemantibiotikum
Composition contenant un pénème ou carbapénème antibiotique

(30) Priority: 15.10.1984 JP 215683/84; 28.01.1985 JP 14001/85
(43) Date of publication of application: 23.04.1986
(73) Proprietor: SANKYO COMPANY LIMITED, Tokyo (JP)
(72) Inventor: Shiogari, Takashi Product Development Lab., Shinagawa-ku Tokyo, 140 (JP); Iwata, Masayuki Product Development Lab., Shinagawa-ku Tokyo, 140 (JP); Ueda, Seigo Product Development Lab., Shinagawa-ku Tokyo, 140 (JP)
(74) Representative: Gibson, Christian John Robert

(56) References cited:
- EP-A- 0 007 614
- EP-A- 0 072 014
- EP-A- 0 091 594

## Description

The present invention relates to a novel composition comprising a penem or carbapenem antibiotic in association with an amino acid derivative. The invention also provides these compounds for treating bacterial infections by administering them to the patient, simultaneously or sequentially.

The class of compounds known as "penem and carbapenem antibiotics" is, of course, very well-known and is potentially of great value for the treatment of bacterial infections. Although, as a group, these penem and carbapenem antibiotics exhibit excellent anti-bacterial activity and a variety of other properties which render them highly suitable for pharmaceutical use, they do have a number of disadvantages. One of the problems of these antibiotics is that, in general, they exhibit a degree of renal toxicity, and some degree of kidney damage is a frequent side effect of their use; accordingly, such penem and carbapenem antibiotics should not be used for the treatment of patients with actual or suspected impaired renal function. As a result, the penem and carbapenem antibiotics cannot be used for many patients for whom otherwise they would be the antibiotic of choice. The problem of renal toxicity is particularly acute when the antibiotics are administered by intravenous or intramuscular injection in a high dose.

We have now surprisingly found that the concurrent, or effectively concurrent, administration, with the penem or carbapenem antibiotic, of one or more of a certain limited class of acylated amino acid derivatives significantly reduces this renal toxicity.

Accordingly, in one aspect, the present invention provides a composition comprising: (a) an antibiotic which is a penem antibiotic or a carbapenem antibiotic; and (b) a pharmaceutically acceptable N-acylated amino acid, in which the amino acid is ornithine, lysine, phenylglycine or phenylalanine, and the acyl group is a C₁-C₁₈ alkanoyl group, a C₃-C₈ alkenoyl group, a C₃-C₈ alkynoyl group, an aromatic acyl group wherein the aryl part is C₆-C₁₄ carbocyclic aryl and is unsubstituted or has from 1 to 5 C₁-C₄ alkyl, hydroxy, C₁-C₄ alkoxy, amino or sulphonyl substituents, a cycloalkanecarbonyl group where in the cycloalkane part is C₃-C₈, a C₂-C₇ alkoxycarbonyl group, or an aralkyloxycarbonyl group where in the aralkyl part has from 7 to 9 carbon atoms and is unsubstituted or has from 1 to 5 amino, C₁₋C₄ alkyl, C₁-C₄ alkoxy or hydroxy substituents; or a pharmaceutically acceptable salt thereof; and wherein the amount of said N-acylated amino acid is sufficient to alleviate any renal toxicity of said antibiotic, preferably in a weight ratio of from 0.1:1 to 4:1.

In another aspect, the invention provides the use of:
(a) an antibiotic which is a penem antibiotic or a carbapenem antibiotic; and
(b) a pharmaceutically acceptable N-acylated derivative of an amino acid, in which the amino acid is ornithine, lysine, phenylglycine or phenylalanine, wherein the acyl group and amount are as defined above, for the manufacture of a one-part or two-part medicament for the treatment of bacterial infections.

The invention also provides a two-part pharmaceutical preparation comprising, in one part, (a) and, in the other part, (b).

There is no particular limitation on the nature of the penem or carbapenem antibiotic to which the present invention can be applied and it is believed that the beneficial effects of the concurrent or effectively concurrent administration of an acylated amino acid derivative will be achieved regardless of the particular antibiotic chosen. However, the antibiotics which are currently of most actual or potential interest may be represented by the general formula I: 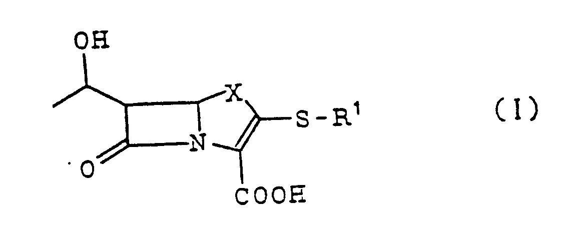
in which:
X represents a sulphur atom, a methylene group or a methylene group having 1 or 2 methyl substituents; and
R¹ represents a group of formula:

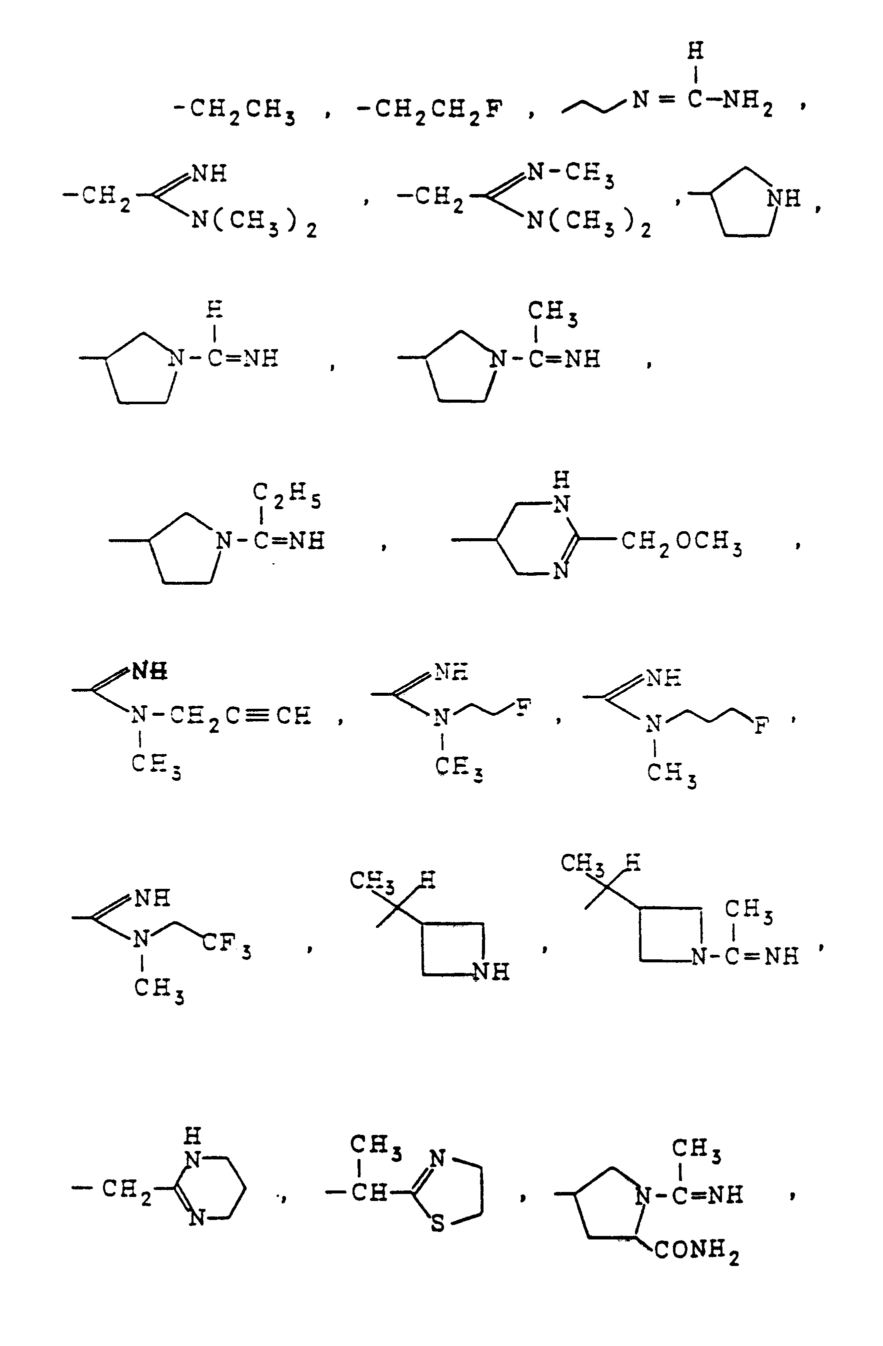

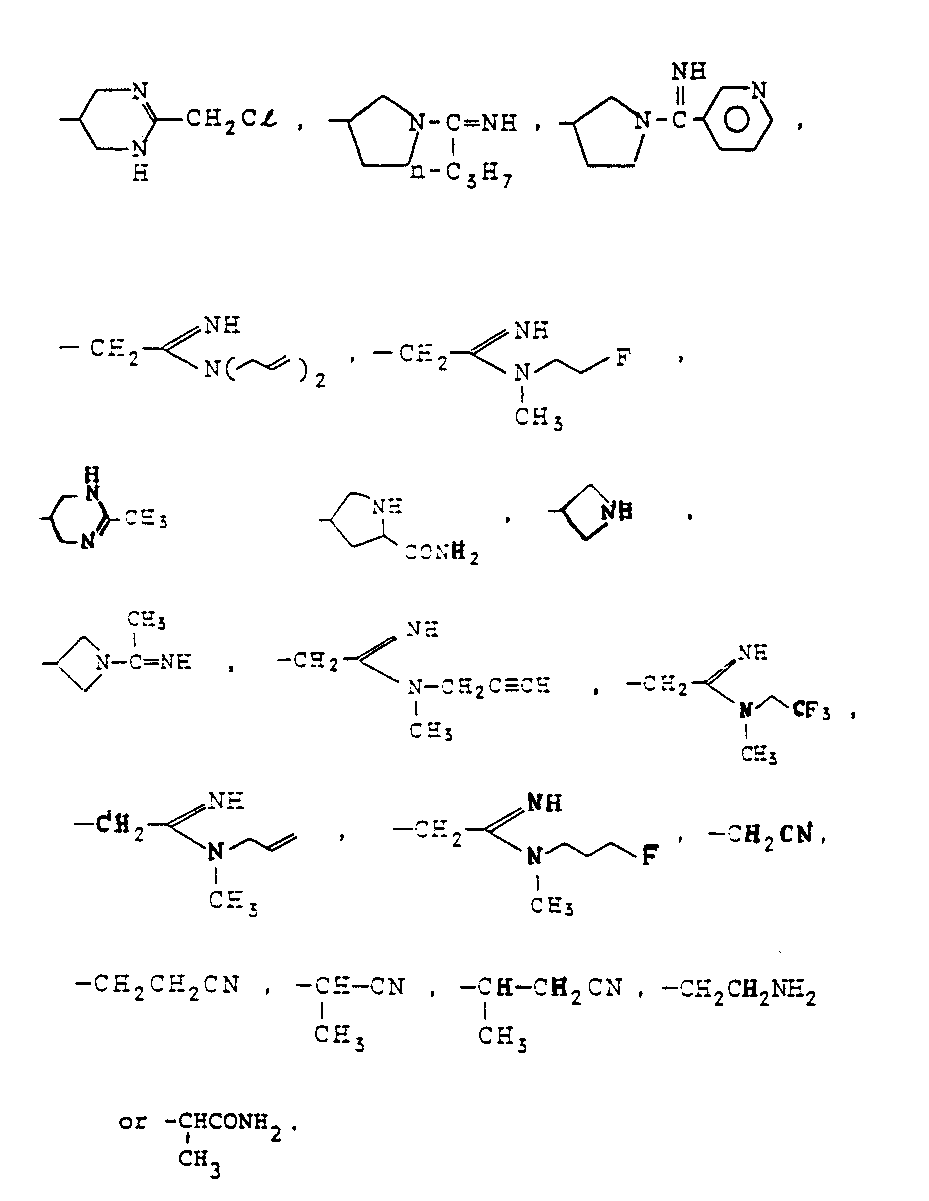

The invention may also be applied to pharmaceutically acceptable salts and esters of such antibiotics.

Specific examples of compounds of general formula I which may be employed in the present invention are those in which R¹ and X are as defined below. The penem and carbapenem compounds are hereinafter, where appropriate, identified by the numbers assigned to them in this list.

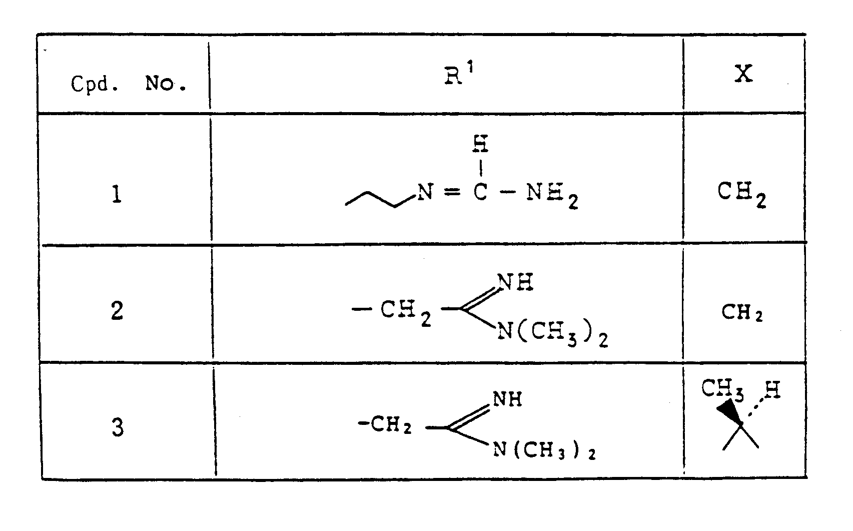

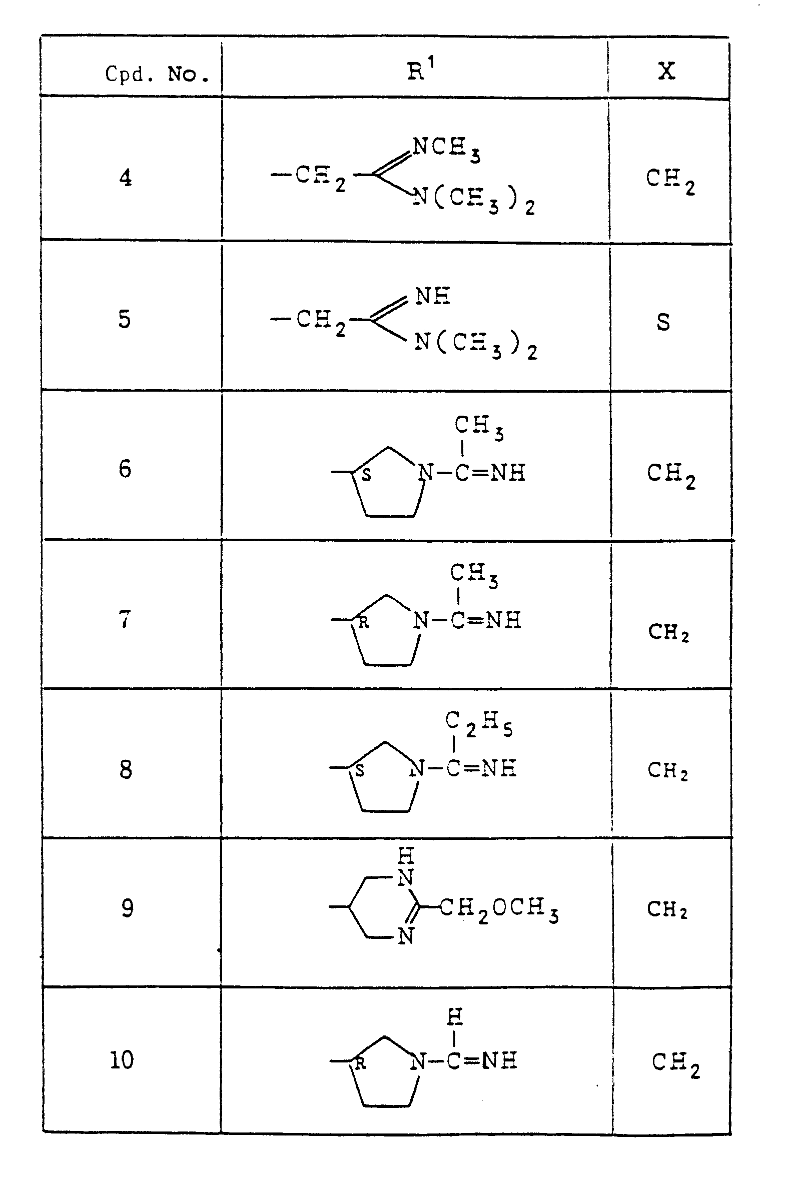

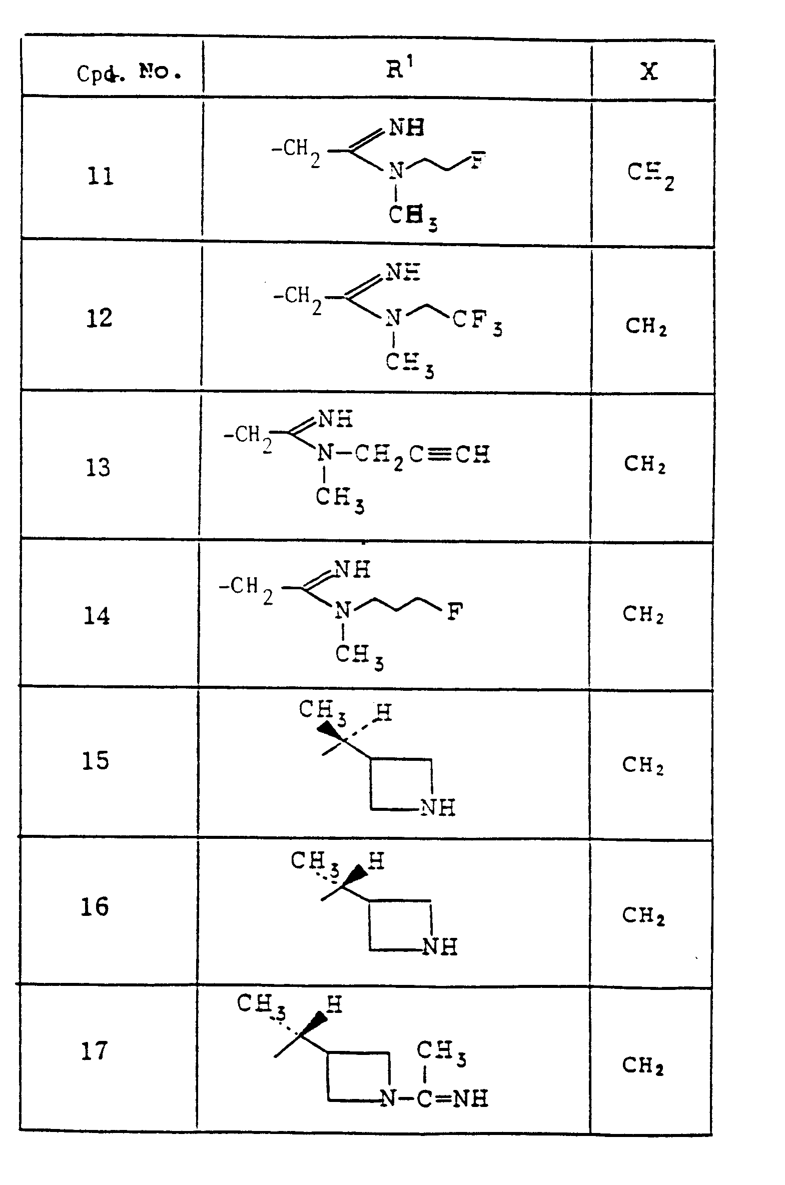

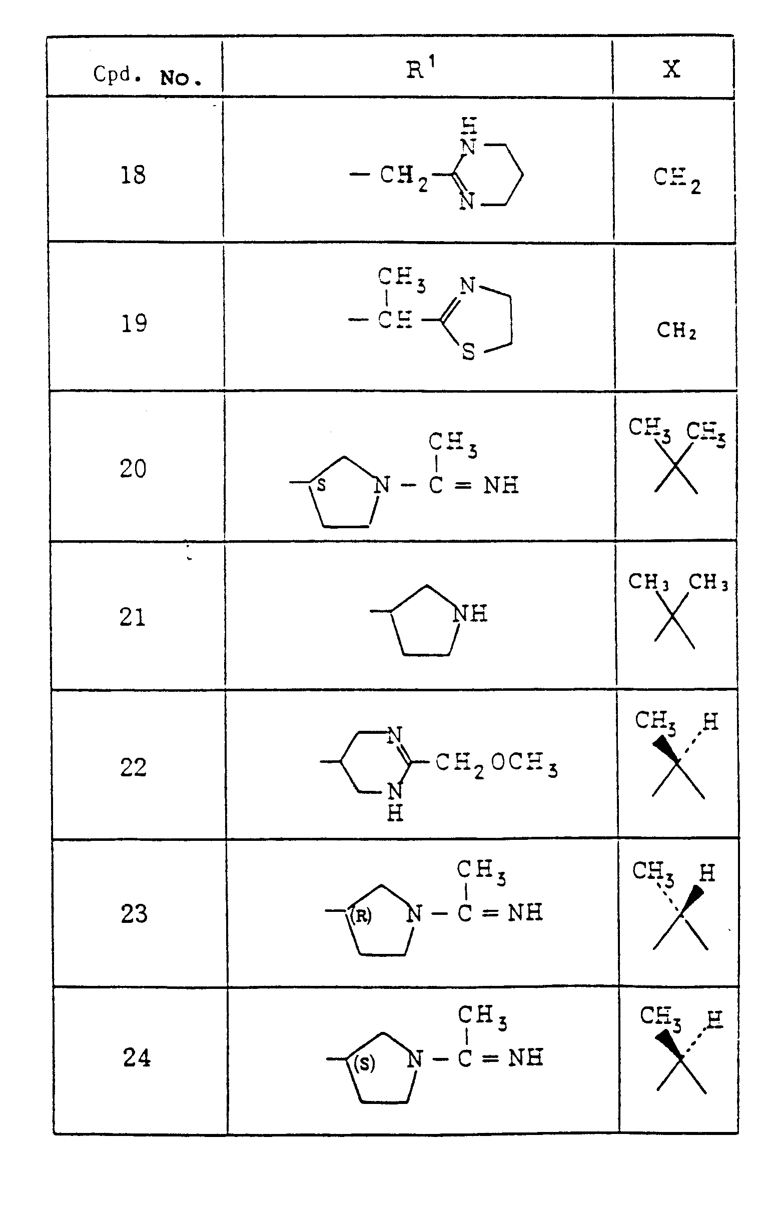

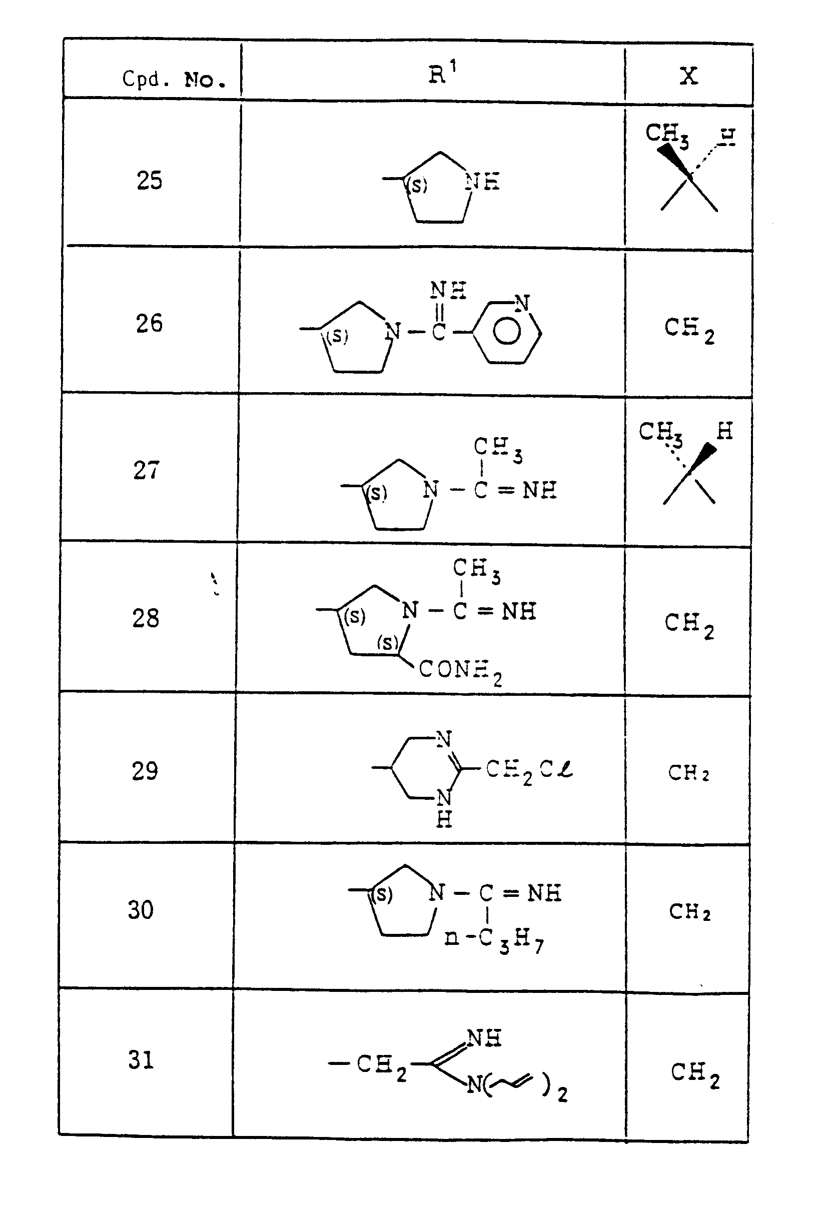

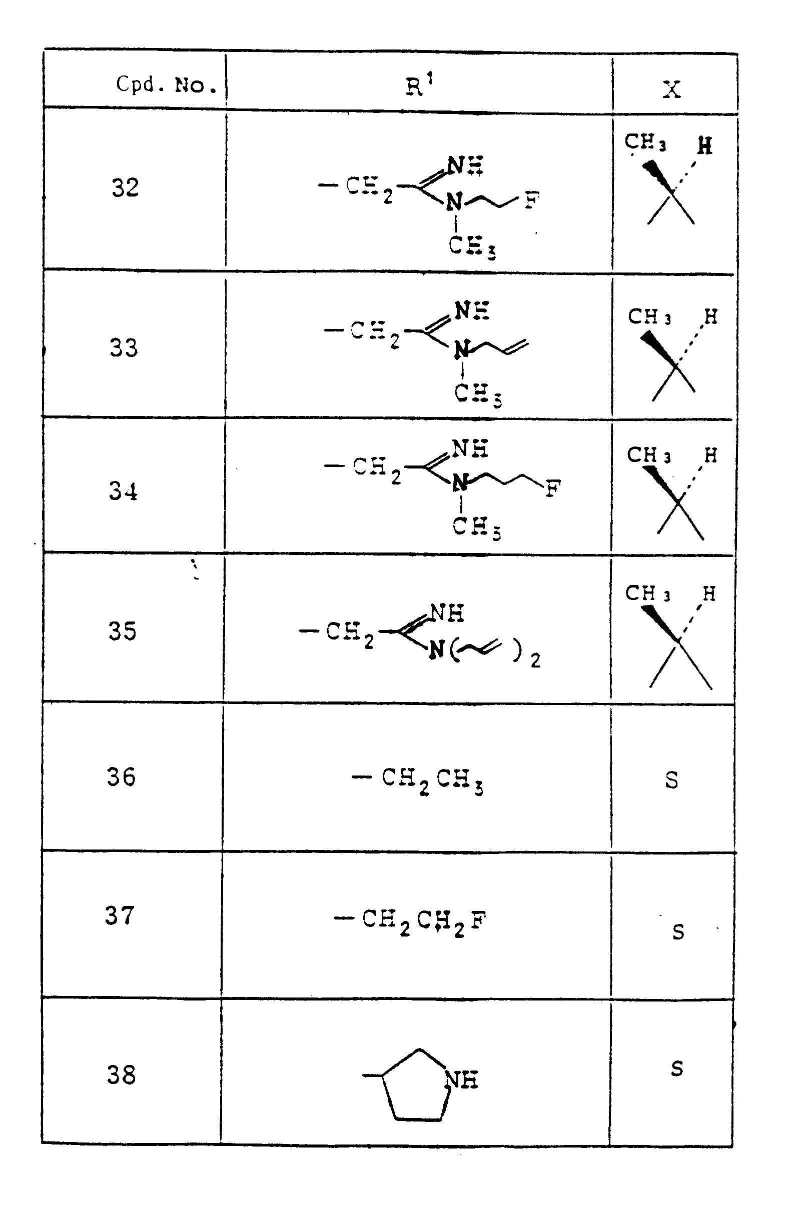

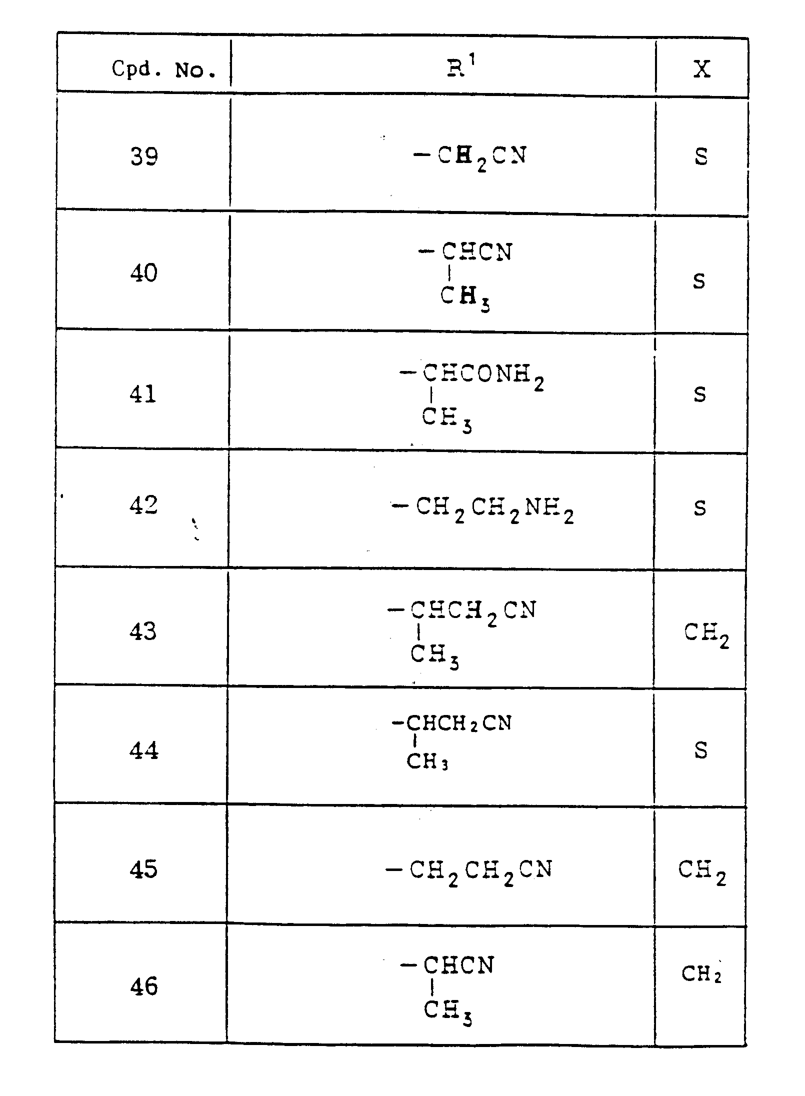

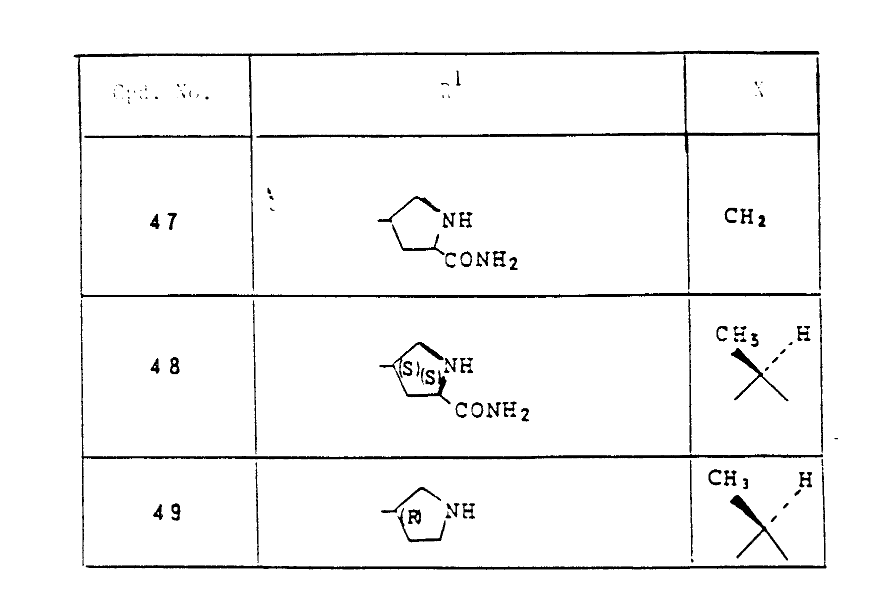

Of the compounds listed above, we particularly prefer those which have the same configuration as thienamycin, i.e. (5R,6S)-6-[1(R)-hydroxyethyl]. In particular, the following compounds are preferred:
(5R,6S)-2-{2-[(aminomethylene)amino]ethylthio}-6-[1(R)-hydroxyethyl]-2-carbapenem-3-carboxylic acid (isomer of Compound No. 1)
(5R,6S)-2-[(3S)-1-acetimidoylpyrrolidin-3-ylthio]-6-[1(R)-hydroxyethyl]-2-carbapenem-3-carboxylic acid (isomer of Compound No. 6)
(5R,6S)-2-[(3R)-1-acetimidoylpyrrolidin-3-ylthio]-6-[1(R)-hydroxyethyl]-2-carbapenem-3-carboxylic acid (isomer of Compound No. 7)
(5R,6S)-2-[(3R)-1-acetimidoylpyrrolidin-3-ylthio]-6-[1(R)-hydroxyethyl]-1(S)-methyl-2-carbapenem-3-carboxylic acid (isomer of Compound No. 23)
(5R,6S)-2-[(3S)-1-acetimidoylpyrrolidin-3-ylthio]-6-[1(R)-hydroxyethyl]-1(R)-methyl-2-carbapenem-3-carboxylic acid (isomer of Compound No. 24)
(5R,6S)-2-[(3S)-1-acetimidoylpyrrolidin-3-ylthio]-6-[1(R)-hydroxyethyl]-1(S)-methyl-2-carbapenem-3-carboxylic acid (isomer of Compound No. 27)
(5R,6S)-2-[(3S)-1-acetimidoyl-5(S)-carbamoylpyrrolidin-3-ylthio]-6-[1(R)-hydroxyethyl]-2-carbapenem-3-carboxylic acid (isomer of Compound No. 28)
The above compounds may likewise be employed in the form of their pharmaceutically acceptable salts or esters, examples of which are well-known to those skilled in the art and which are given, for example, in EP-A-00 72710.

N-acylated derivatives of ornithine and lysine may be represented by the general formula II:

R²-NH-(CH₂)ₙ-CH(COOH)NH-R³ (II)

and phenylalanine and phenylglycine derivatives may be represented by the general formula III:

Ph-(CH₂)ₘ-CH(COOH)-NHR⁴ (III)

In the above formulae, n represents the integer 3 or 4, whilst m represents the cypher 0 or the integer 1.

Ph represents the phenyl group.

R² and R³ are the same or different and each represents a hydrogen atom or a carboxylic acyl group as defined, provided that R² and R³ are not simultaneously hydrogen atoms.

R⁴ represents a carboxylic acyl group.

Examples of carboxylic acyl groups which may be represented by R², R³ and R⁴ include:
alkanoyl groups having from 1 to 18, preferably from 2 to 5, carbon atoms, for example the acetyl, propionyl, butyryl, isobutyryl, valeryl, isovaleryl, pivaloyl, hexanoyl, heptanoyl, octanoyl, nonanoyl and decanoyl groups;
alkenoyl and alkynoyl groups having from 3 to 8, more preferably 3 or 4, carbon atoms, for example the acryloyl, methacryloyl, crotonoyl or propioloyl groups;
aromatic acyl groups in which the aryl ring is a carbocyclic ring having from 6 to 14, preferably 6 to 10, carbon atoms and optionally having from 1 to 5, more preferably from 1 to 3, C₁-C₄ alkyl, hydroxy, alkoxy, amino or sulphonyl substituents, for example the benzoyl and naphthoyl groups and the benzoyl and naphthoyl groups having one or more of the above substituents;
alicyclic acyl groups in which the carbocyclic ring has from 3 to 8 carbon atoms, more preferably 3 or 6 carbon atoms, for example the cyclopropanecarbonyl and cyclohexanecarbonyl groups;
alkoxycarbonyl groups having a total of from 2 to 7 carbon atoms, for example the methoxycarbonyl, ethoxycarbonyl, propoxycarbonyl, isopropoxycarbonyl, butoxycarbonyl and pentyloxycarbonyl groups; and
aralkyloxycarbonyl groups in which the aralkyl moiety has from 7 to 9 carbon atoms and is unsubstituted or has from 1 to 5, more preferably from 1 to 3, amino, C₁-C₄ alkyl, C₁-C₄ alkoxy or hydroxy substituents, for example the benzyloxycarbonyl, phenethyloxycarbonyl, 3-phenylpropoxycarbonyl, 4-methoxybenzyloxycarbonyl, 4-hydroxybenzyloxycarbonyl, p-tolyloxycarbonyl and 4-aminobenzyloxycarbonyl groups.

Of the groups exemplified above, the following are preferred: acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl and 4-methoxybenzyloxycarbonyl groups, of which the acetyl and benzoyl, particularly benzoyl, groups are most preferred.

Specific examples of the amino acid compounds which may be employed in the present invention are given in the following list. It should, of course, be appreciated that these compounds can exist in the D-, L- and DL- forms and that any of these forms can be employed in the present invention. The compounds are hereinafter referred to by the numbers appended to them in this list. In the case of the mono-acylated derivatives of ornithine, the acyl group can be present on either the N^{α}- or the N^{δ}-position, whilst, in the case of mono-acylated derivatives of lysine, the acyl group can be present on either the N^{α}- or N^{ε}-position. Of course, the respective individual mono-acylated derivatives or a mixture of the N^{α} and N^{δ} or N^{α} and N^{ε} derivatives may be used.
When the compounds of the invention are hereinafter referred to by the numbers appended to them in the following list, the N^{α} isomer is identified by the appropriate number followed by the character "α", the N^{δ} isomer is identified by the number followed by "δ" and the N^{ε} isomer is identified by the number followed by "ε". Where the number alone is given, a mixture is meant. Where the compound is identified by the number followed by "α/δ" or "α/ε", this defines specifically the three options: the isolated N^{α} isomer; the isolated N^{δ} isomer or N^{ε} isomer; or a mixture of these two isomers.
1. N-Acetylornithine
2. N-Propionylornithine
3. N-Butyrylornithine
4. N-Isobutyrylornithine
5. N-Valerylornithine
6. N-Hexanoylornithine
7. N-Heptanoylornithine
8. N-Octanoylornithine
9. N-Nonanoylornithine
10. N-Decanoylornithine
11. N-Crotonoylornithine
12. N-Benzoylornithine
13. N-Cyclopropanecarbonylornithine
14. N-Cyclohexanecarbonylornithine
15. N^{α},N^{δ}-Diacetylornithine
16. N^{α},N^{δ}-Dipropionylornithine
17. N^{α},N^{δ}-Dibutyrylornithine
18. N^{α},N^{δ}-Diisobutyrylornithine
19. N^{α},N^{δ}-Divalerylornithine
20. N^{α},N^{δ}-Diisovalerylornithine
21. N^{α},N^{δ}-Dihexanoylornithine
22. N^{α},N^{δ}-Diheptanoylornithine
23. N^{α},N^{δ}-Dioctanoylornithine
24. N^{α},N^{δ}-Dinonanoylornithine
25. N^{α},N^{δ}-Didecanoylornithine
26. N^{α},N^{δ}-Dicrotonoylornithine
27. N^{α},N^{δ}-Dibenzoylornithine
28. N^{α},N^{δ}-Dicyclopropanecarbonylornithine
29. N^{α},N^{δ}-Dicyclohexanecarbonylornithine
30. N^{α}-Isobutyryl-N^{δ}-benzoylornithine
31. N^{α}-Crotonoyl-N^{δ}-benzoylornithine
32. N^{α}-Cyclopropanecarbonyl-N^{δ}-isobutyrylornithine
33. N^{α}-Cyclohexanecarbonyl-N^{δ}-crotonoylornithine
34. N^{α}-Benzoyl-N^{δ}-isobutyrylornithine
35. N^{α}-Benzoyl-N^{δ}-crotonoylornithine
36. N^{α}-Benzoyl-N^{δ}-cyclopropanecarbonylornithine
37. N^{α}-Benzoyl-N^{δ}-cyclohexanecarbonylornithine
38. N^{α}-Benzoyl-N^{δ}-acetylornithine
39. N^{α}-Benzoyl-N^{δ}-propionylornithine
40. N-Methoxycarbonylornithine
41. N-Ethoxycarbonylornithine
42. N-Butoxycarbonylornithine
43. N^{α},N^{δ}-Dimethoxycarbonylornithine
44. N^{α},N^{δ}-Diethoxycarbonylornithine
45. N^{α},N^{δ}-Dibutoxycarbonylornithine
46. N^{α}-Methoxycarbonyl-N^{δ}-ethoxycarbonylornithine
47. N^{α}-Methoxycarbonyl-N^{δ}-butoxycarbonylornithine
48. N^{α}-Ethoxycarbonyl-N^{δ}-methoxycarbonylornithine
49. N^{α}-Butoxycarbonyl-N^{δ}-ethoxycarbonylornithine
50. N^{α}-Ethoxycarbonyl-N^{δ}-butyrylornithine
51. N^{α}-Ethoxycarbonyl-N^{δ}-crotonoylornithine
52. N^{α}-Ethoxycarbonyl-N^{δ}-cyclopropanecarbonylornithine
53. N^{α}-Ethoxycarbonyl-N^{δ}-benzoylornithine
54. N^{α}-Propionyl-N^{δ}-ethoxycarbonylornithine
55. N^{α}-Benzoyl-N^{δ}-ethoxycarbonylornithine
56. N-Acetyllysine
57. N-Propionyllysine
58. N-Butyryllysine
59. N-Isobutyryllysine
60. N-Valeryllysine
61. N-Hexanoyllysine
62. N-Heptanoyllysine
63. N-Octanoyllysine
64. N-Nonanoyllysine
65. N-Decanoyllysine
66. N-Crotonoyllysine
67. N-Benzoyllysine
68. N-Cyclopropanecarbonyllysine
69. N-Cyclohexanecarbonyllysine
70. N^{α},N^{ε}-Diacetyllysine
71. N^{α},N^{ε}-Dipropionyllysine
72. N^{α},N^{ε}-Dibutyryllysine
73. N^{α},N^{ε}-Diisobutyryllysine
74. N^{α},N^{ε}-Divaleryllysine
75. N^{α},N^{ε}-Diisovaleryllysine
76. N^{α},N^{ε}-Dihexanoyllysine
77. N^{α},N^{ε}-Diheptanoyllysine
78. N^{α},N^{ε}-Dioctanoyllysine
79. N^{α},N^{ε}-Dinonanoyllysine
80. N^{α},N^{ε}-Didecanoyllysine
81. N^{α},N^{ε}-Dicrotonoyllysine
82. N^{α},N^{ε}-Dibenzoyllysine
83. N^{α},N^{ε}-Dicyclopropanecarbonyllysine
84. N^{α},N^{ε}-Dicyclohexanecarbonyllysine
85. N^{α}-Isobutyryl-N^{ε}-crotonoyllysine
86. N^{α}-Isobutyryl-N^{ε}-benzoyllysine
87. N^{α}-Crotonoyl-N^{ε}-benzoyllysine
88. N^{α}-Cyclopropanecarbonyl-N^{ε}-isobutyryllysine
89. N^{α}-Cyclohexanecarbonyl-N^{ε}-crotonoyllysine
90. N^{α}-Cyclopropanecarbonyl-N^{ε}-cyclohexanecarbonyllysine
91. N^{α}-Cyclohexanecarbonyl-N^{ε}-benzoyllysine
92. N^{α}-Benzoyl-N^{ε}-isobutyryllysine
93. N^{α}-Benzoyl-N^{ε}-crotonoyllysine
94. N^{α}-Benzoyl-N^{ε}-cyclopropanecarbonyllysine
95. N^{α}-Benzoyl-N^{ε}-cyclohexanecarbonyllysine
96. N^{α}-Benzoyl-N^{ε}-acetyllysine
97. N^{α}-Benzoyl-N^{ε}-propionyllysine
98. N-Methoxycarbonyllysine
99. N-Ethoxycarbonyllysine
100. N-Butoxycarbonyllysine
101. N^{α},N^{ε}-Dimethoxycarbonyllysine
102. N^{α},N^{ε}-Diethoxycarbonyllysine
103. N^{α},N^{ε}-Dibutoxycarbonyllysine
104. N^{α}-Methoxycarbonyl-N^{ε}-ethoxycarbonyllysine
105. N^{α}-Methoxycarbonyl-N^{ε}-butoxycarbonyllysine
106. N^{α}-Ethoxycarbonyl-N^{ε}-methoxycarbonyllysine
107. N^{α}-Butoxycarbonyl-N^{ε}-ethoxycarbonyllysine
108. N^{α}-Ethoxycarbonyl-N^{ε}-propionyllysine
109. N^{α}-Ethoxycarbonyl-N^{ε}-crotonoyllysine
110. N^{α}-Ethoxycarbonyl-N^{ε}-cyclohexanecarbonyllysine
111. N^{α}-Ethoxycarbonyl-N^{ε}-benzoyllysine
112. N^{α}-Isobutyryl-N^{ε}-ethoxycarbonyllysine
113. N^{α}-Benzoyl-N^{ε}-ethoxycarbonyllysine
114. N-Acetylphenylglycine
115. N-Benzoylphenylglycine
116. N-Acetylphenylalanine
117 N-Benzoylphenylalanine
118. N-Propionylphenylglycine
119. N-Butyrylphenylglycine
120. N-Isobutyrylphenylglycine
121. N-Valerylphenylglycine
122. N-Hexanoylphenylglycine
123. N-Heptanoylphenylglycine
124. N-Octanoylphenylglycine
125. N-Nonanoylphenylglycine
126. N-Decanoylphenylglycine
127. N-Crotonoylphenylglycine
128. N-Cyclopropanecarbonylphenylglycine
129. N-Cyclohexanecarbonylphenylglycine
130. N-Methoxycarbonylphenylglycine
131. N-Ethoxycarbonylphenylglycine
132. N-Butoxycarbonylphenylglycine
133. N-Propionylphenylalanine
134. N-Butyrylphenylalanine
135. N-Isobutyrylphenylalanine
136. N-Valerylphenylalanine
137. N-Hexanoylphenylalanine
138. N-Heptanoylphenylalanine
139. N-Octanoylphenylalanine
140. N-Nonanoylphenylalanine
141. N-Decanoylphenylalanine
142. N-Crotonoylphenylalanine
143. N-Cyclopropanecarbonylphenylalanine
144. N-Cyclohexanecarbonylphenylalanine
145. N-Methoxycarbonylphenylalanine
146. N-Ethoxycarbonylphenylalanine
147. N-Butoxycarbonylphenylalanine
148. N-(4-Hydroxybenzoyl)lysine
149. N-Anisoyllysine
150. N-(4-Aminobenzoyl)lysine
151. N-Naphthoyllysine
152. N-(4-Sulphonylbenzoyl)lysine
153. N-Toluoyllysine
154. N-Benzyloxycarbonyllysine
155. N-(4-Methoxybenzyloxycarbonyl)lysine
156. N^{α},N^{ε}-Di(4-hydroxybenzoyl)lysine
157. N^{α},N^{ε}-Dianisoyllysine
158. N^{α},N^{ε}-Di(4-aminobenzoyl)lysine
159. N^{α},N^{ε}-Dinaphthoyllysine
160. N^{α}-Cyclohexanecarbonyl-N^{ε}-acetyllysine
161. N^{α},N^{ε}-Ditoluoyllysine
162. N^{α},N^{ε}-Dibenzyloxycarbonyllysine
163. N^{α},N^{ε}-Di(4-methoxybenzyloxycarbonyl)lysine
164. N-(4-Hydroxybenzoyl)ornithine
165. N-Anisoylornithine
166. N-(4-Aminobenzoyl)ornithine
167. N-Naphthoylornithine
168. N-(4-Sulphonylbenzoyl)ornithine
169. N-Toluoylornithine
170. N-Benzyloxycarbonylornithine
171. N-(4-Methoxybenzyloxycarbonyl)ornithine
172. N^{α},N^{δ}-Di(4-hydroxybenzoyl)ornithine
173. N^{α},N^{δ}-Dianisoylornithine
174. N^{α},N^{δ}-Di(4-aminobenzoyl)ornithine
175. N^{α},N^{δ}-Dinaphthoylornithine
176. N^{α}-Cyclohexanecarbonyl-N^{δ}-acetylornithine
177. N^{α},N^{δ}-Ditoluoylornithine
178. N^{α},N^{δ}-Dibenzyloxycarbonylornithine
179. N^{α},N^{δ}-Di(4-methoxybenzyloxycarbonyl)ornithine
180. N-(4-Hydroxybenzoyl)phenylglycine
181. N-Anisoylphenylglycine
182. N-(4-Aminobenzoyl)phenylglycine
183. N-Naphthoylphenylglycine
184. N-(4-Sulphonylbenzoyl)phenylglycine
185. N-Toluoylphenylglycine
186. N-Benzyloxycarbonylphenylglycine
187. N-(4-Methoxybenzyloxycarbonyl)phenylglycine
188. N-(4-Hydroxybenzoyl)phenylalanine
189. N-(4-Sulphonylbenzoyl)phenylalanine
190. N-(4-Aminobenzoyl)phenylalanine
191. N-Naphthoylphenylalanine
192. N-Anisoylphenylalanine
193. N-Toluoylphenylalanine
194. N-Benzyloxycarbonylphenylalanine
195. N-(4-Methoxybenzyloxycarbonyl)phenylalanine

Of the amino acid derivatives listed above, the following are particularly preferred: Compounds No. 27, 29, 37, 38, 82, 84, 95, 96, 115, 117, 129, 144, 160 and 176.

The amino acid derivatives employed in the present invention are acids and, as such, are capable of forming salts; any pharmaceutically acceptable salt of these amino acids may be employed. Examples of such salts include: alkali metal salts such as the sodium or potassium salts; alkaline earth metal salts, such as the calcium salt; other metal salts, such as the magnesium, aluminium, iron, zinc, copper, nickel and cobalt salts; the ammonium salt; and salts with amino sugars, such as glucosamine and galactosamine.

The compositions of the invention may be prepared by any suitable method which involves mixing the antibiotic with the amino acid derivative and the invention is not intended to be limited by any particular method of preparation. Since the amino acid derivatives employed in this invention have, in general, very limited solubility in water, it is preferred that they should first be dispersed in water and then converted to a suitable salt by adding an aqueous solution of an appropriate base, for example: a metal compound, such as sodium hydroxide or potassium hydroxide; ammonia; or an amino sugar, such as glucosamine or galactosamine. Sufficient of the base is added to adjust the pH of the mixture to a value within the range from 6 to 9.

The penem or carbapenem antibiotic is then added to the resulting solution. The mixed solution thus obtained may be employed as such or it may first be lyophilized to give a powdery mixture, which may be subsequently formulated into an appropriate dosage form suitable for the chosen route of administration, either by the manufacturer or prior to use.

Although it is convenient to administer the antibiotic and the amino acid derivative simultaneously to a patient in a single composition, it is, of course, clear that the two compounds may be administered separately, provided that they are administered sufficiently closely in time to each other that the amino acid derivative has a suitable concentration in the blood for all or most of the time that the antibiotic is present. Normally, it is anticipated that this will be achieved if the two compounds are administered within one hour of each other.

The composition of the invention is particularly suitable for use by intravenous administration.

There is no particular restriction on the relative proportions of the amino acid derivative and the penem or carbapenem antibiotic; in general, we have found that proportions of amino acid derivative to antibiotic of from 0.1:1 to 4:1 give good results, but equally proportions outside this range may successfully be employed.

The invention is further illustrated by the following Examples and Activity Tests. In the following, the penem or carbapenem antibiotics are referred to by the numbers appended to them in the foregoing list and are identified as "Penem Cpd No." whilst the amino acid derivatives are also identified by the numbers appended to them in the foregoing list and are referred to as "Amino Acid Cpd No."

### EXAMPLE 1

5 g of N^{α},N^{ε}-dibenzoyllysine were weighed out and dispersed in 80 ml of water. A 1N aqueous solution of sodium hydroxide was slowly added to this dispersion, and the N^{α},N^{ε}-dibenzoyllysine dissolved when the pH of the solution reached a value of 7-8. Then, 5 g of (5R,6S)-2-[(3S)-1-acetimidoylpyrrolidin-3-ylthio]-6-[1(R)-hydroxyethyl]-2-carbapenem-3-carboxylic acid (Penem Compound No. 6) were dissolved in this solution, to give a total volume of 100 ml.

### EXAMPLE 2

Similar procedures to those described in Example 1 were carried out, using the other penem or carbapenem antibiotic substances and the other ornithine, lysine phenylglycine or phenylalanine derivatives shown in the following Table, in the amounts shown in that Table.

In this Table, where the amino acid derivatives are not specified as being D- or L-, then they are the DL-form.

### ACTIVITY TESTS

The preparation obtained by the procedure described in Example 1 was injected into the ear vein of a rabbit (about 3 kg body weight) in an amount of 3 ml/kg [that is 150 mg/kg of the penem Compound No. 6 + 150 mg/kg of N^{α},N^{ε}-dibenzoyllysine (amino acid Compound No. 82)]. A preparation which had been obtained by the same procedure as that described in Example 1 but not including the N^{α},N^{ε}-dibenzoyllysine was injected into another rabbit, as a control, in a similar manner to the above.

After one week, the kidneys of both rabbits were excised and examined. A change was observed in the renal tissue of the rabbits to which had been administered the preparation without the N^{α},N^{ε}-dibenzoyllysine, but no such change was observed at all in the renal tissue of rabbits to which had been administered the preparation containing the N^{α,}N^{ε}-dibenzoyllysine.

Similar experiments were carried out on other preparations which were prepared from other penem or carbapenem antibiotic substances and other amino acid derivatives. The Table also shows the results of these experiments.

The observed change in the renal tissue of the control rabbits was a degenerative necrosis of the proximal renal tubules in the region of the renal cortex. In the following Table, where the proportion of the total area of this region which exhibited such necrosis was from 0 to 25%, this is shown as +++. Where the area is less than 50% this is shown as ++ and where it is less than 75% this is shown as +. Where different animals within each test group exhibited different responses, this is shown as e.g. +++ - ++ or ++ - +.

It should be noted that, whenever penem or carbapenem antibiotic substances which were not combined with amino acid derivatives were administered, a change in the renal tissue was observed.

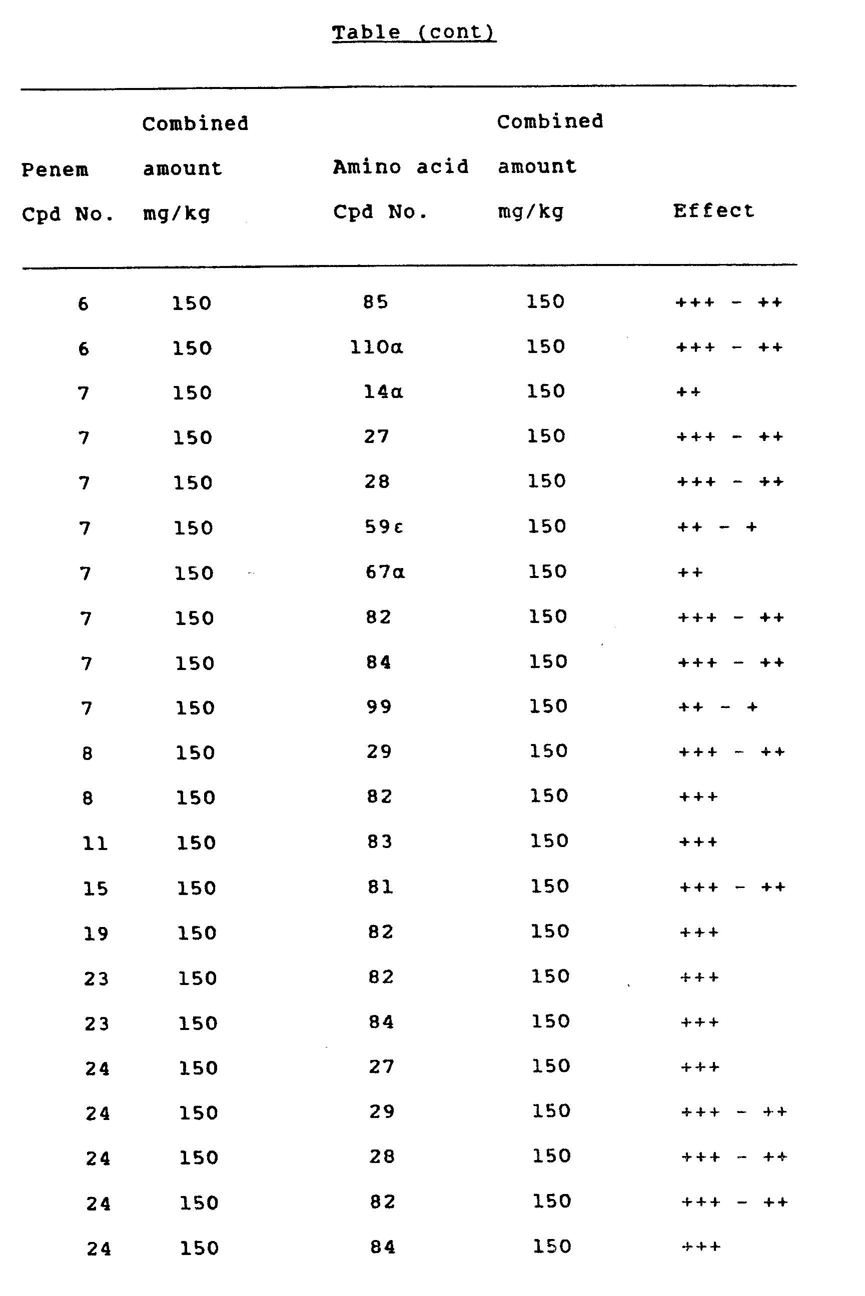

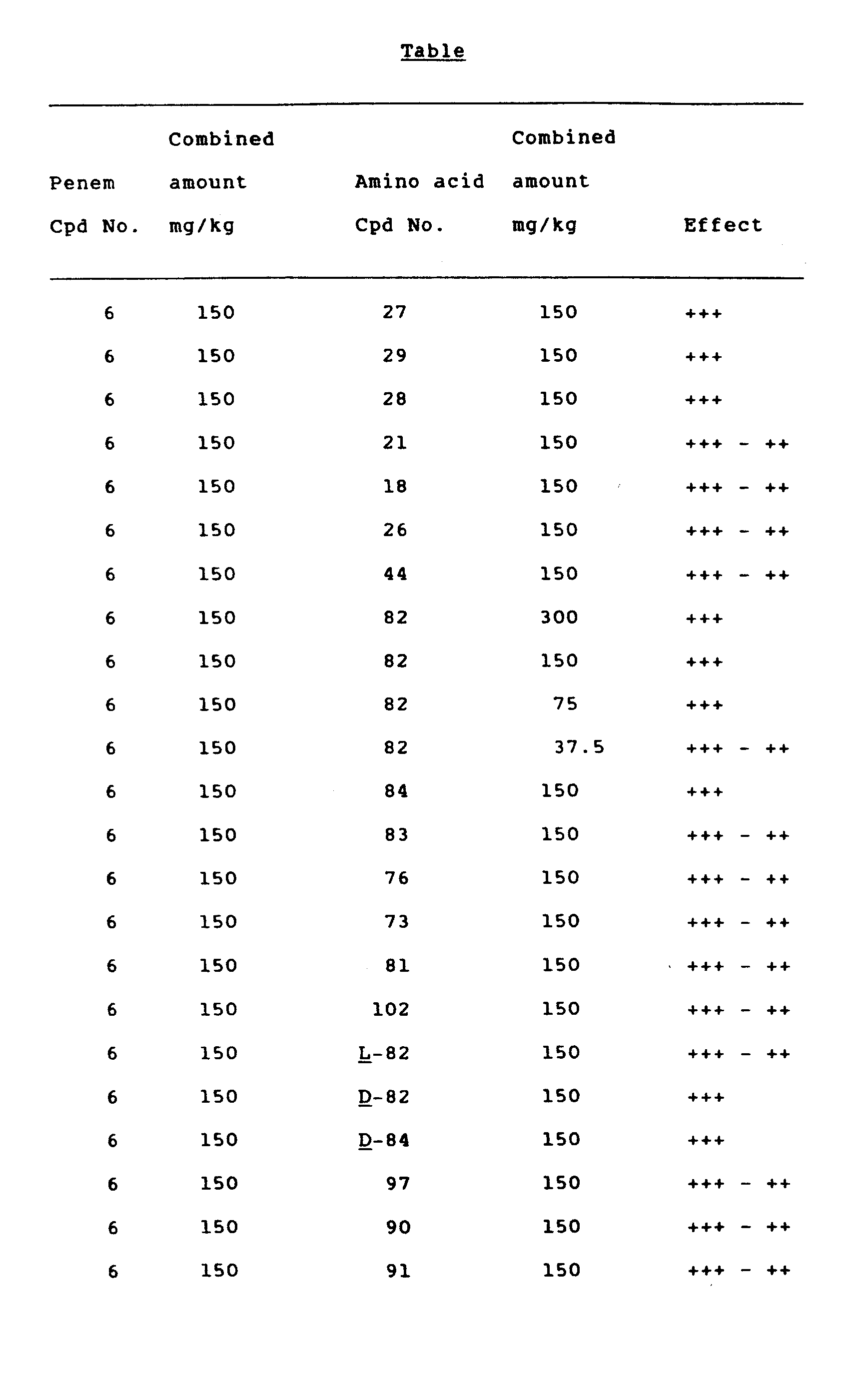

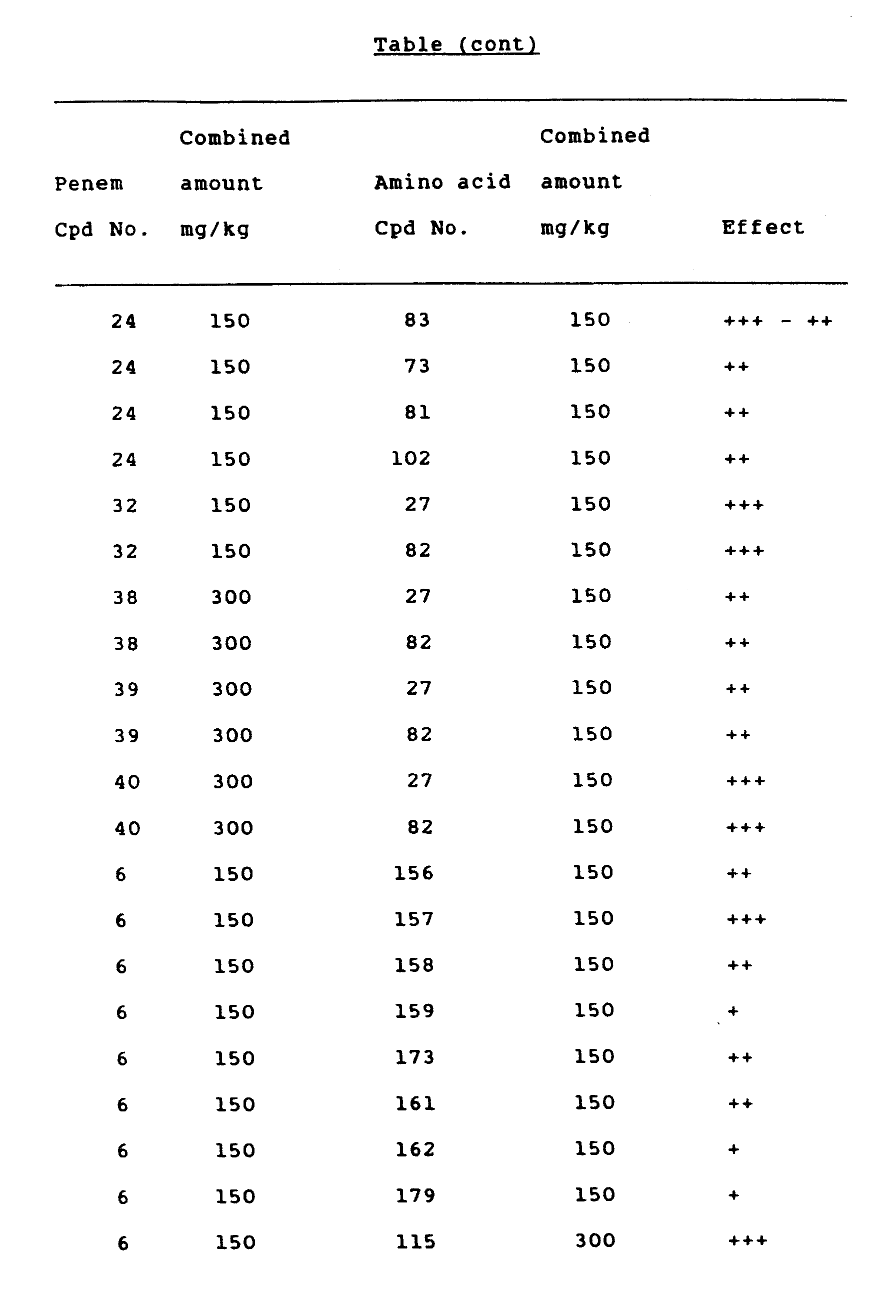

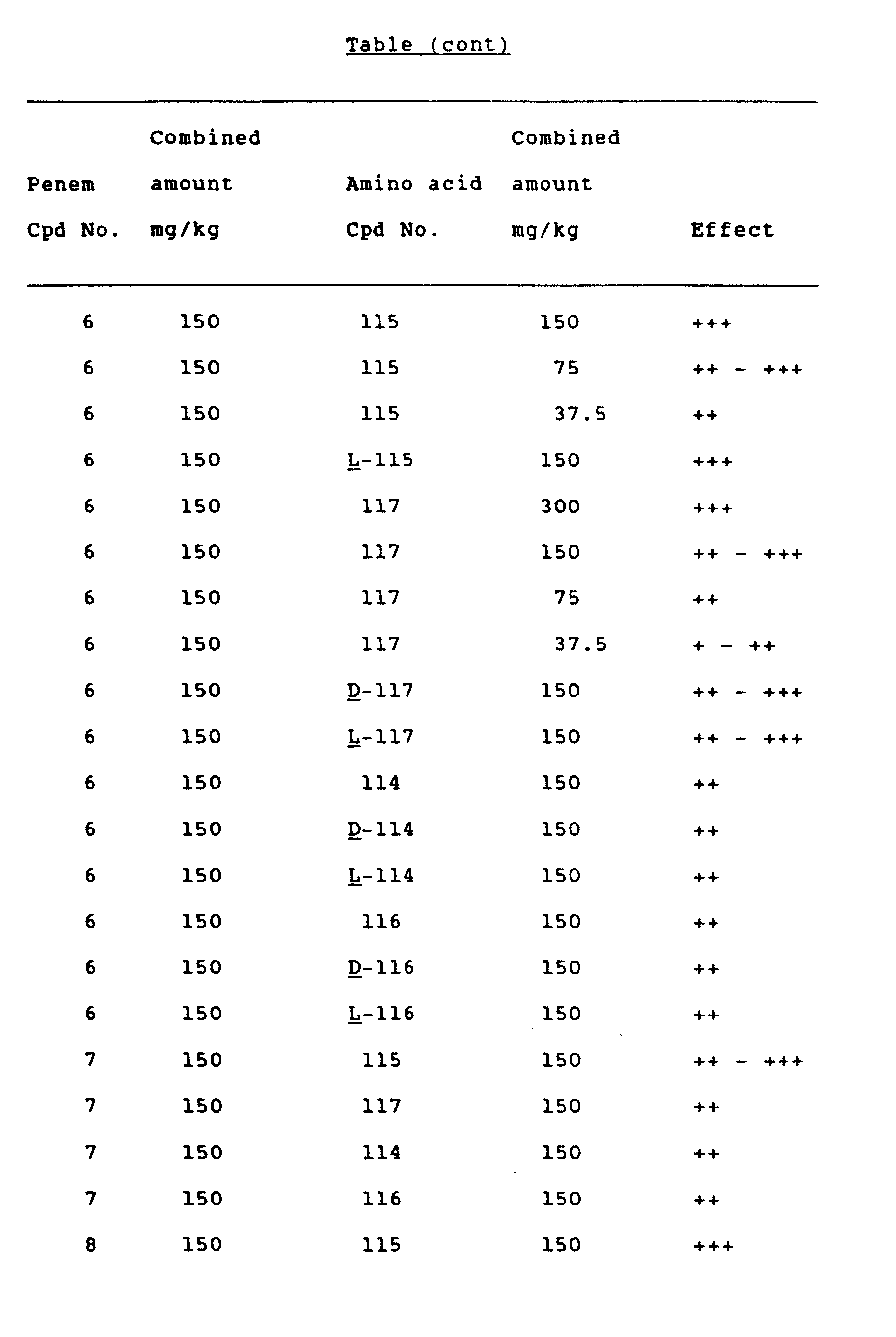

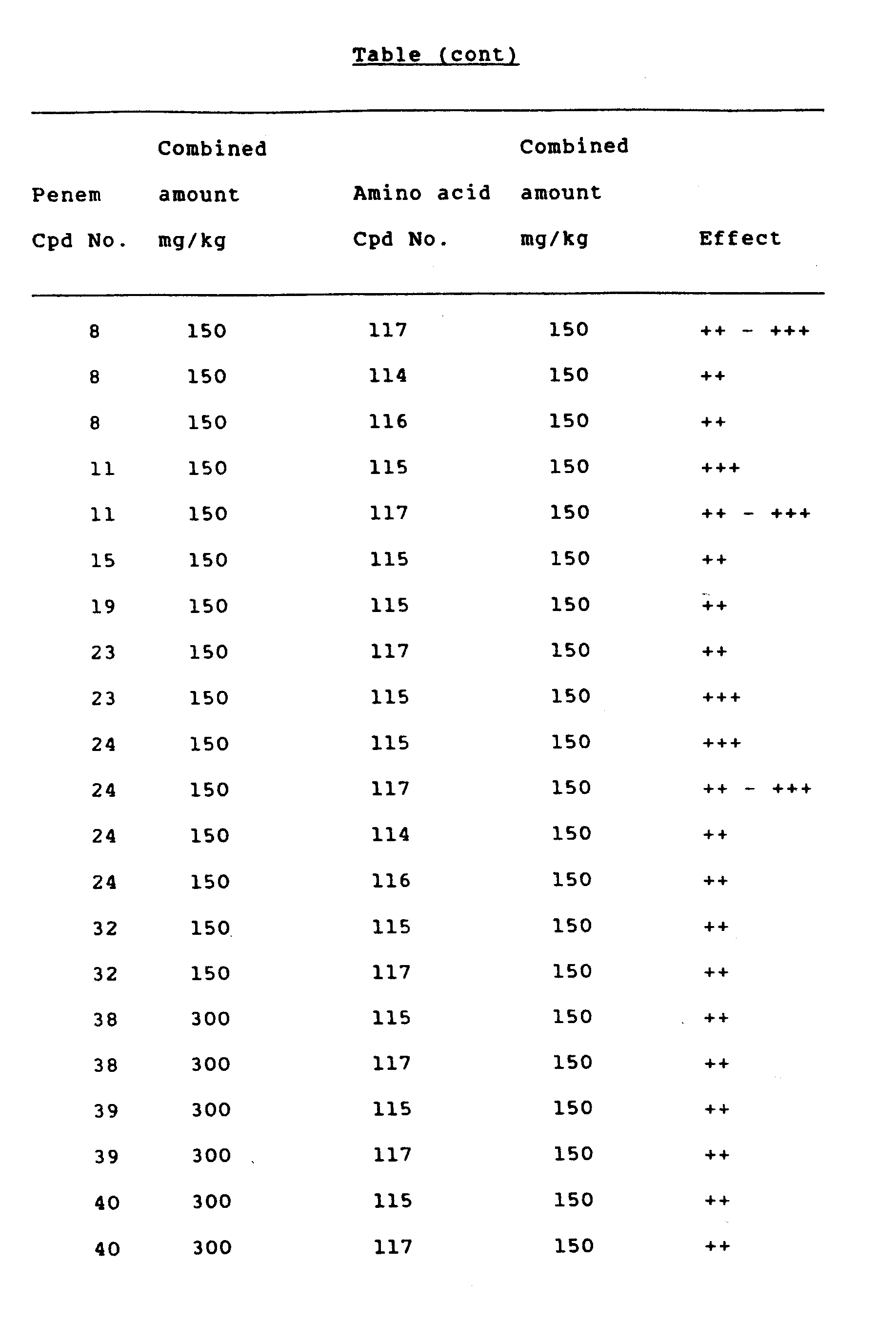

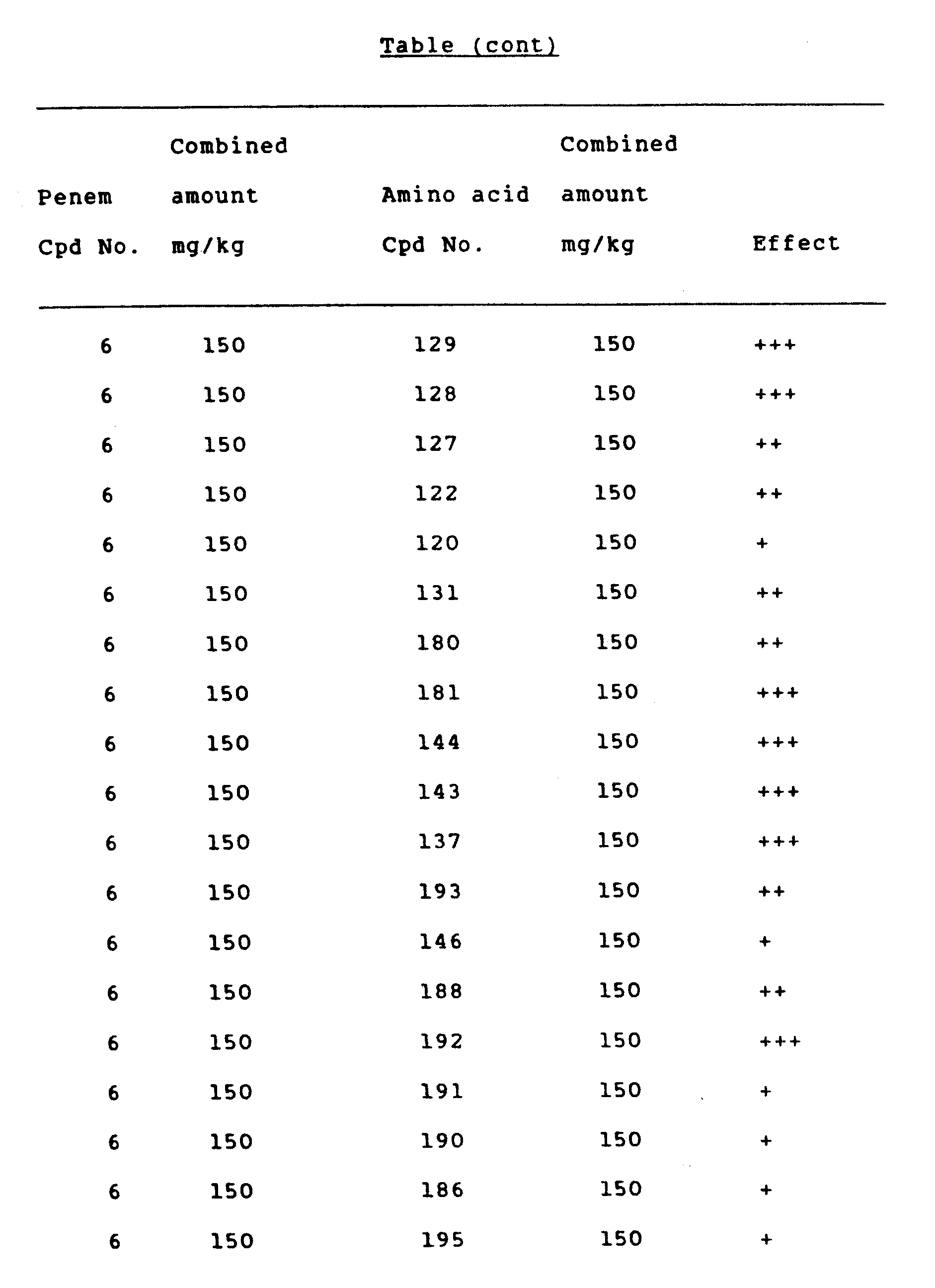

## Claims (Claims for the following Contracting State(s): BE, CH, DE, FR, GB, IT, LI, NL, SE)

1. A composition comprising: (a) an antibiotic which is a penem antibiotic or a carbapenem antibiotic; and (b) a pharmaceutically acceptable N-acylated amino acid, in which the amino acid is ornithine, lysine, phenylglycine or phenylalanine, and the acyl group is a C₁-C₁₈ alkanoyl group, a C₃-C₈ alkenoyl group, a C₃-C₈ alkynoyl group, an aromatic acyl group wherein the aryl part is C₆-C₁₄ carbocyclic aryl and is unsubstituted or has from 1 to 5 C₁-C₄ alkyl, hydroxy, C₁-C₄ alkoxy, amino or sulphonyl substituents, a cycloalkanecarbonyl group wherein the cycloalkane part is C₃-C₈, a C₂-C₇ alkoxycarbonyl group, or an aralkyloxycarbonyl group wherein the aralkyl part has from 7 to 9 carbon atoms and is unsubstituted or has from 1 to 5 amino, C₁-C₄ alkyl, C₁-C₄ alkoxy or hydroxy substituents; or a therapeutically acceptable salt thereof; and wherein the amount of said N-acylated amino acid is sufficient to alleviate any renal toxicity of said antibiotic, preferably in a weight ratio of from 0.1:1 to 4:1.

2. A composition as claimed in Claim 1, wherein said N-acylated amino acid is a compound of general formula II:
R²-NH-(CH₂)ₙ-CH(COOH)-NHR³ (II)
wherein:
n is 3 or 4; and
R² and R³ are the same or different and each represents a hydrogen atom or a carboxylic acyl group, provided that R² and R³ are not both hydrogen atoms;
wherein the carboxylic acyl group is a C₁-C₁₈ alkanoyl group, a C₃-C₈ alkenoyl group, a C₃-C₈ alkynoyl group, an aromatic acyl group wherein the aryl part is C₆-C₁₄ carbocyclic aryl and is unsubstituted or has from 1 to 5 C₁-C₄ alkyl, hydroxy, C₁-C₄ alkoxy, amino or sulphonyl substituents, a cycloalkanecarbonyl group wherein the cycloalkane part is C₃-C₈, a C₂-C₇ alkoxycarbonyl group, or an aralkyloxycarbonyl group wherein the aralkyl part has from 7 to 9 carbon atoms and is unsubstituted or has from 1 to 5 amino, C₁-C₄ alkyl, C₁-C₄ alkoxy or hydroxy substituents;
or a pharmaceutically acceptable salt thereof.

3. A composition as claimed in Claim 2, wherein R² and R³ are the same or different and each represents a hydrogen atom or an acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl or 4-methoxybenzyloxycarbonyl group, provided that R² and R³ are not both hydrogen atoms.

4. A composition as claimed in Claim 2, wherein at least one of R² and R³ represents an acetyl and/or benzoyl group.

5. A composition as claimed in Claim 1, wherein said N-acylated amino acid is a compound of general formula III:
Ph-(CH₂)ₘ-CH(COOH)-NHR⁴ (III)
wherein:
Ph represents a phenyl group;
m is 0 or 1; and
R⁴ represents a carboxylic acyl group as defined;
or a pharmaceutically acceptable salt thereof.

6. A composition as claimed in Claim 5, wherein R⁴ represents an acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl or 4-methoxybenzyloxycarbonyl group.

7. A composition as claimed in Claim 5, wherein R⁴ represents an acetyl or benzoyl group.

8. A composition as claimed in Claim 1, wherein said N-acylated amino acid is:
N^{α},N^{δ}-Dibenzoylornithine
N^{α},N^{δ}-Dicyclohexanecarbonylornithine
N^{α}-Benzoyl-N^{δ}-cyclohexanecarbonylornithine
N^{α}-Benzoyl-N^{δ}-acetylornithine
N^{α},N ^{ε}-Dibenzoyllysine
N^{α},N^{ε}-Dicyclohexanecarbonyllysine
N^{α}-Benzoyl-N^{ε}-cyclohexanecarbonyllysine
N^{α}-Benzoyl-N^{ε}-acetyllysine
N-Benzoylphenylglycine
N-Benzoylphenylalanine
N-Cyclohexanecarbonylphenylglycine
N-Cyclohexanecarbonylphenylalanine
N^{α}-Cyclohexanecarbonyl-N^{ε}-acetyllysine
or
N^{α}-Cyclohexanecarbonyl-N^{δ}-acetylornithine.

9. A composition as claimed in any one of Claims 1 to 8, wherein said antibiotic is a compound of general formula I: 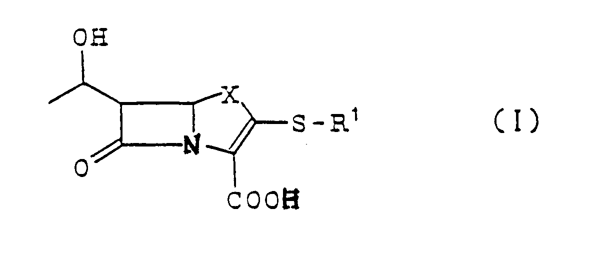 wherein:
X represents a sulphur atom, a methylene group, or a methylene group having 1 or 2 methyl substituents;
and
R¹ represents a group of formula: 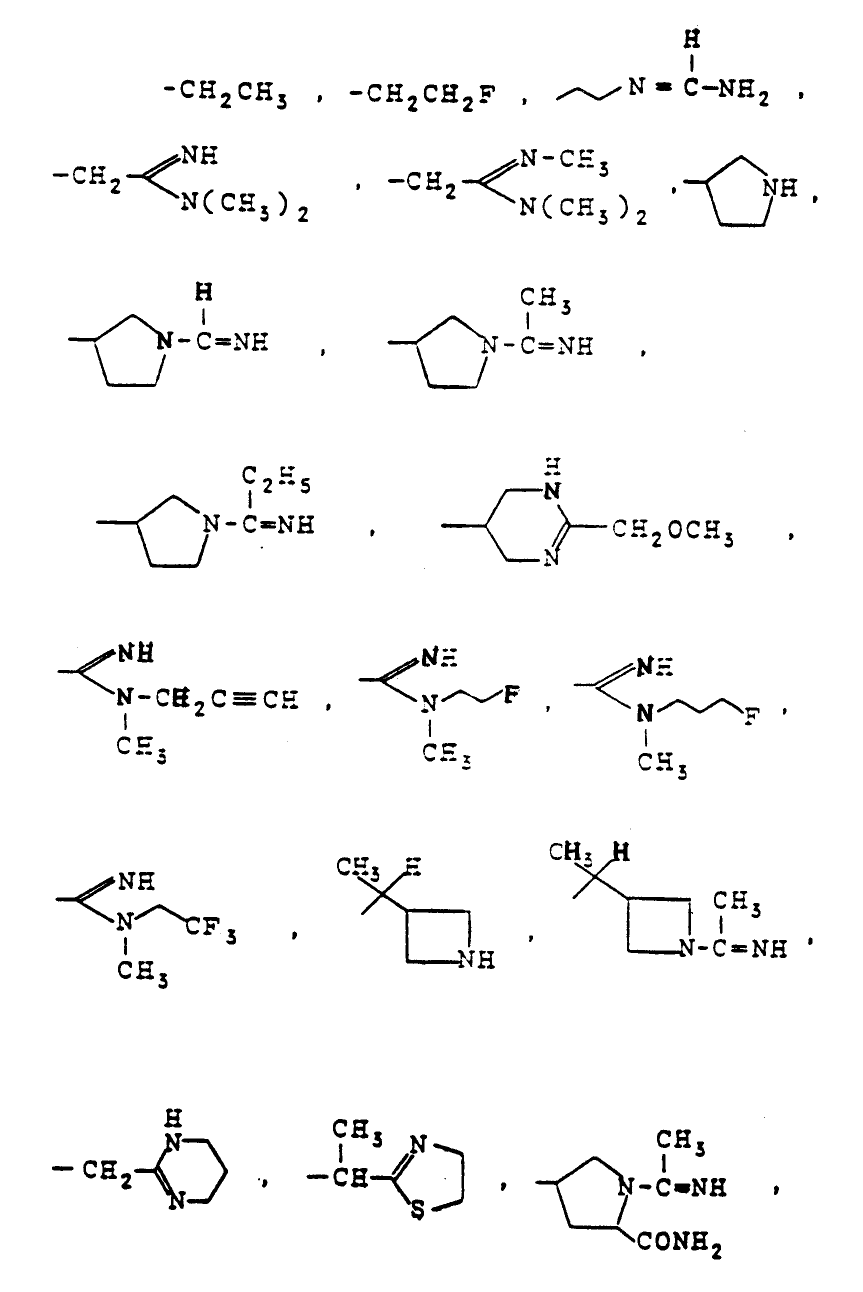 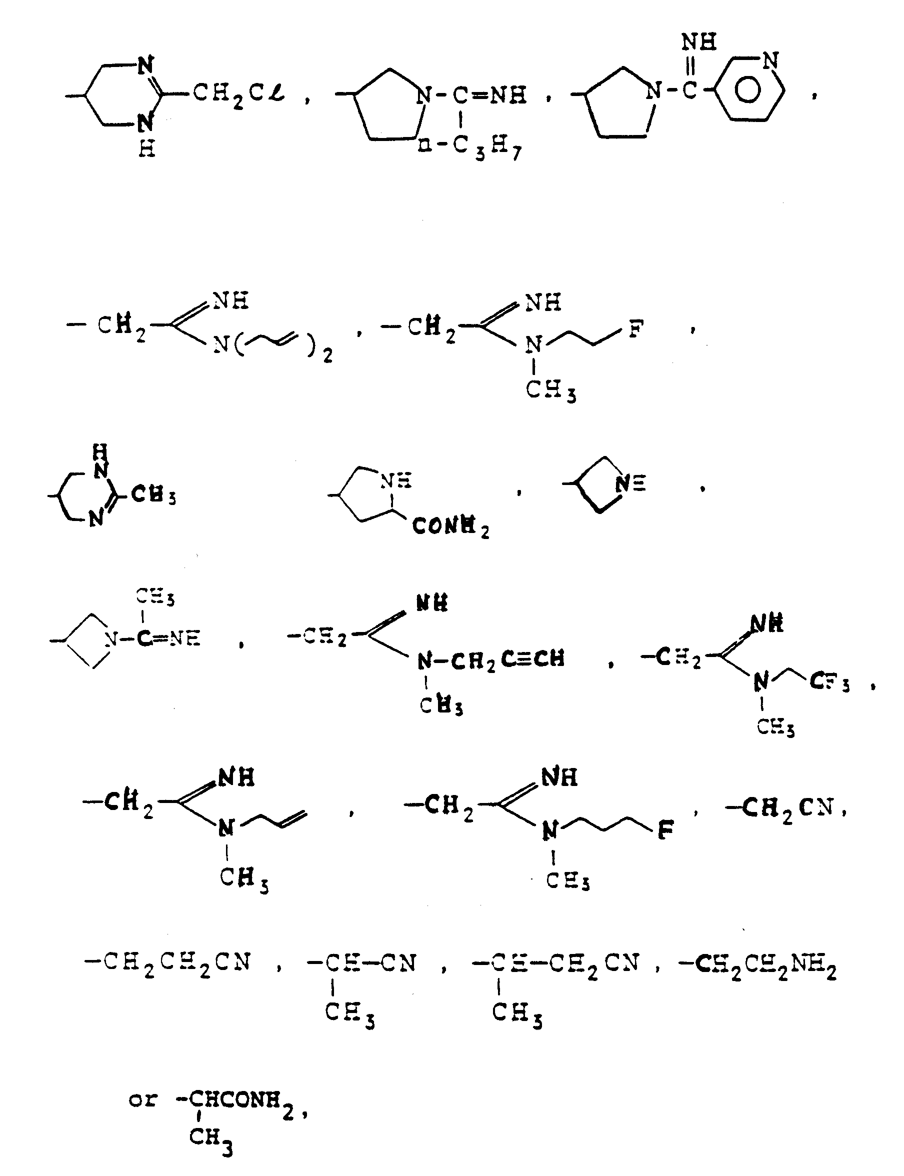 or a pharmaceutically acceptable salt thereof.

10. A composition as claimed in Claim 9, wherein said antibiotic is:
(5R,6S)-2-{2-[(aminomethylene)amino]ethylthio}-6-[1(R)-hydroxyethyl]-2-carbapenem-3-carboxylic acid
(5R,6S)-2-[(3S)-1-acetimidoylpyrrolidin-3-ylthio]-6-[1(R)-hydroxyethyl]-2-carbapenem-3-carboxylic acid
(5R,6S)-2-[(3R)-1-acetimidoylpyrrolidin-3-ylthio]-6-[1(R)-hydroxyethyl]-2-carbapenem-3-carboxylic acid
(5R,6S)-2-[(3R)-1-acetimidoylpyrrolidin-3-ylthio]-6-[1(R)-hydroxyethyl]-1(S)-methyl-2-carbapenem-3-carboxylic acid
(5R,6S)-2-[(3S)-1-acetimidoylpyrrolidin-3-ylthio]-6-[1(R)-hydroxyethyl]-1(R)-methyl-2-carbapenem-3-carboxylic acid
(5R,6S)-2-[(3S)-1-acetimidoylpyrrolidin-3-ylthio]-6-[1(R)-hydroxyethyl]-1(S)-methyl-2-carbapenem-3-carboxylic acid
or
(5R,6S)-2-[(3S)-1-acetimidoyl-5(S)-carbamoylpyrrolidin-3-ylthio]-6-[1(R)-hydroxyethyl]-2-carbapenem-3-carboxylic acid

11. A composition as claimed in any one of Claims 1 to 10, wherein the weight ratio of said N-acylated amino acid to said antibiotic is from 0.1:1 to 4:1.

12. The use of:
(a) a penem antibiotic or a carbapenem antibiotic; and
(b) a pharmaceutically acceptable N-acylated derivative of an amino acid, the amino acid being ornithine, lysine, phenylglycine and phenylalanine, wherein the acyl group and amount are as defined in claim 1, for the manufacture of a one-part or two-part medicament for the treatment of bacterial infections.

13. The use as claimed in Claim 12, wherein said N-acylated amino acid is as defined in any one of Claims 2 to 8.

14. The use as claimed in Claim 12 or Claim 13, wherein said antibiotic is as defined in any one of Claims 9 and 10.

15. The use as claimed in any one of Claims 12 to 14, wherein the weight ratio of said N-acylated amino acid to said antibiotic is from 0.1:1 to 4:1.

16. A packaged pharmaceutical preparation for treating a mammal suffering from a bacterial infection, comprising:
(a) in one part, a penem antibiotic or a carbapenem antibiotic; and
(b) in another part, a pharmaceutically acceptable N-acylated derivative of an amino acid, the amino acid being ornithine, lysine, phenylglycine and phenylalanine, and wherein the acyl group and amount are as defined in claim 1.

17. A preparation as claimed in Claim 16, wherein said N-acylated amino acid is as defined in any one of Claims 2 to 10.

18. A preparation as claimed in Claim 16 or Claim 17, wherein said antibiotic is as defined in any one of Claims 9 and 10.

19. A preparation as claimed in any one of Claims 16 to 18, wherein the weight ratio of said N-acylated amino acid to said antibiotic is from 0.1:1 to 4:1.

## Claims (Claims for the following Contracting State(s): AT)

1. A method of making a pharmaceutical composition by mixing: (a) an antibiotic which is a penem antibiotic or a carbapenem antibiotic; and (b) a pharmaceutically acceptable N-acylated amino acid, in which the amino acid is ornithine, lysine, phenylglycine or phenylalanine, and the acyl group is a C₁-C₁₈ alkanoyl group, a C₃-C₈ alkenoyl group, a C₃-C₈ alkynoyl group, an aromatic acyl group wherein the aryl part is C₆-C₁₄ carbocyclic aryl and is unsubstituted or has from 1 to 5 C₁-C₄ alkyl, hydroxy, C₁-C₄ alkoxy, amino or sulphonyl substituents, a cycloalkanecarbonyl group wherein the cycloalkane part is C₃-C₈, a C₂-C₇ alkoxycarbonyl group, or an aralkyloxycarbonyl group wherein the aralkyl part has from 7 to 9 carbon atoms and is unsubstituted or has from 1 to 5 amino, C₁-C₄ alkyl, C₁-C₄ alkoxy or hydroxy substituents; or a pharmaceutically acceptable salt thereof; and wherein the amount of said N-acylated amino acid is sufficient to alleviate any renal toxicity of said antibiotic, preferably in a weight ratio of from 0.1:1 to 4:1.

2. A method as claimed in Claim 1, wherein said N-acylated amino acid is a compound of general formula II:
R²-NH-(CH₂)ₙ-CH(COOH)-NHR³ (II)
wherein:
n is 3 or 4; and
R² and R³ are the same or different and each represents a hydrogen atom or a carboxylic acyl group, provided that R² and R³ are not both hydrogen atoms;
wherein the carboxylic acyl group is a C₁-C₁₈ alkanoyl group, a C₃-C₈ alkenoyl group, a C₃-C₈ alkynoyl group, an aromatic acyl group wherein the aryl part is C₆-C₁₄ carbocyclic aryl and is unsubstituted or has from 1 to 5 C₁-C₄ alkyl, hydroxy, C₁-C₄ alkoxy, amino or sulphonyl substituents, a cycloalkanecarbonyl group wherein the cycloalkane part is C₃-C₈, a C₂-C₇ alkoxycarbonyl group, or an aralkyloxycarbonyl group wherein the aralkyl part has from 7 to 9 carbon atoms and is unsubstituted or has from 1 to 5 amino, C₁-C₄ alkyl, C₁-C₄ alkoxy or hydroxy substituents;
or a pharmaceutically acceptable salt thereof.

3. A method as claimed in Claim 2, wherein R² and R³ are the same or different and each represents a hydrogen atom or an acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl or 4-methoxybenzyloxycarbonyl group, provided that R² and R³ are not both hydrogen atoms.

4. A method as claimed in Claim 2, wherein at least one of R² and R³ represents an acetyl and/or benzoyl group.

5. A method as claimed in Claim 1, wherein said N-acylated amino acid is a compound of general formula III:
Ph-(CH₂)ₘ-CH(COOH)-NHR⁴ (III)
wherein:
Ph represents a phenyl group;
m is 0 or 1; and
R⁴ represents a carboxylic acyl group as defined;
or a pharmaceutically acceptable salt thereof.

6. A method as claimed in Claim 5, wherein R⁴ represents an acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl or 4-methoxybenzyloxycarbonyl group.

7. A method as claimed in Claim 5, wherein R⁴ represents an acetyl or benzoyl group.

8. A method as claimed in Claim 1, wherein said N-acylated amino acid is:
N^{α},N^{δ}-Dibenzoylornithine
N^{α},N^{δ}-Dicyclohexanecarbonylornithine
N^{α}-Benzoyl-N^{δ}-cyclohexanecarbonylornithine
N^{α}-Benzoyl-N^{δ}-acetylornithine
N^{α},N^{ε}-Dibenzoyllysine
N^{α},N^{ε}-Dicyclohexanecarbonyllysine
N^{α}-Benzoyl-N^{ε}-cyclohexanecarbonyllysine
N^{α}-Benzoyl-N^{ε}-acetyllysine
N-Benzoylphenylglycine
N-Benzoylphenylalanine
N-Cyclohexanecarbonylphenylglycine
N-Cyclohexanecarbonylphenylalanine
N^{α}-Cyclohexanecarbonyl-N^{ε}-acetyllysine
or
N^{α}-Cyclohexanecarbonyl-N^{δ}-acetylornithine.

9. A method as claimed in any one of Claims 1 to 8, wherein said antibiotic is a compound of general formula I: 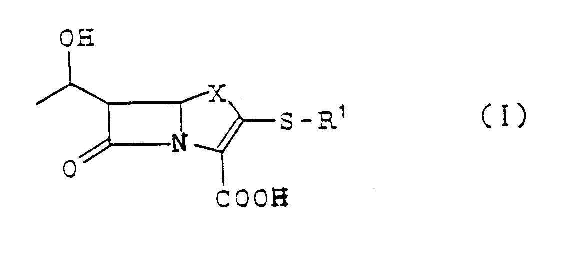 wherein:
X represents a sulphur atom, a methylene group, or a methylene group having 1 or 2 methyl substituents;
and
R¹ represents a group of formula: 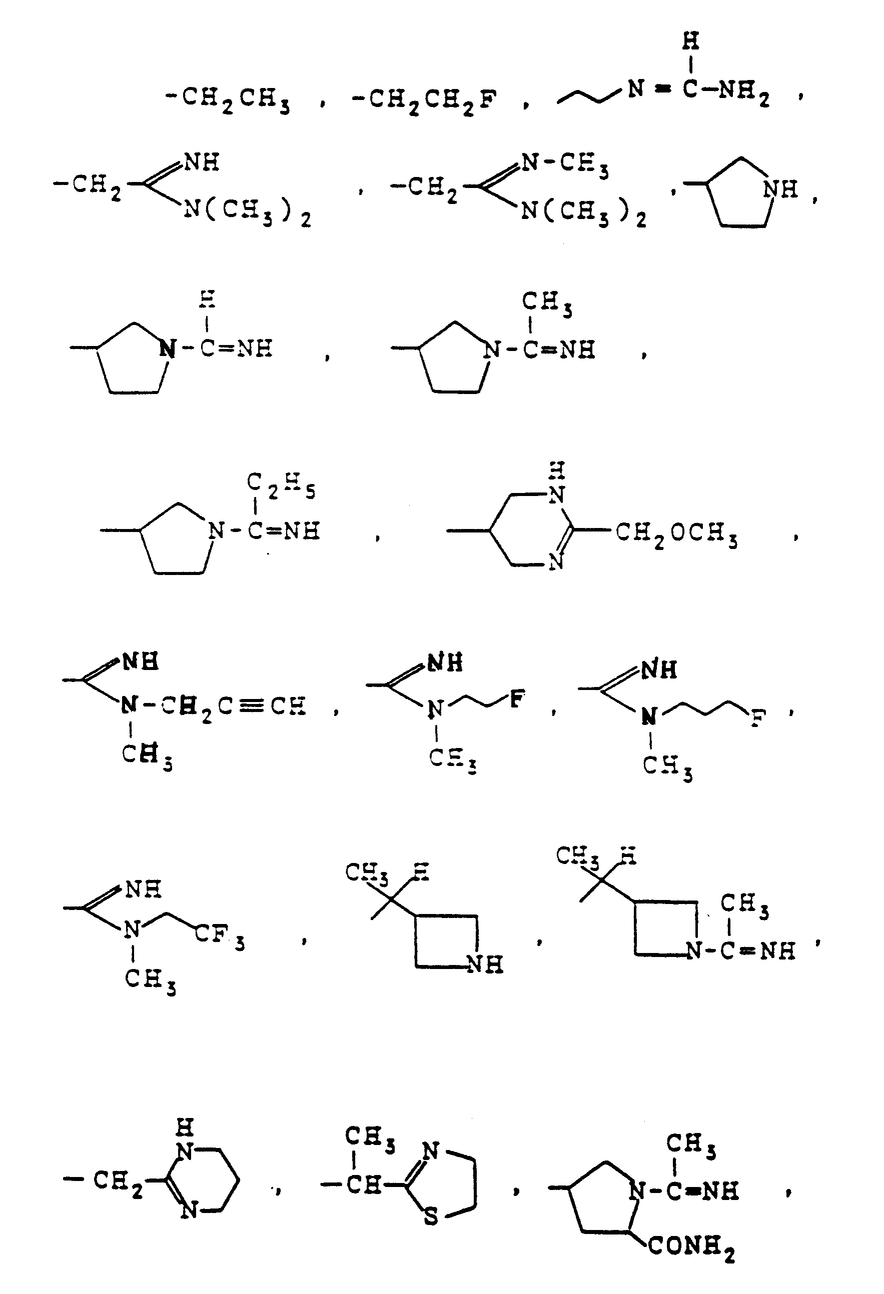 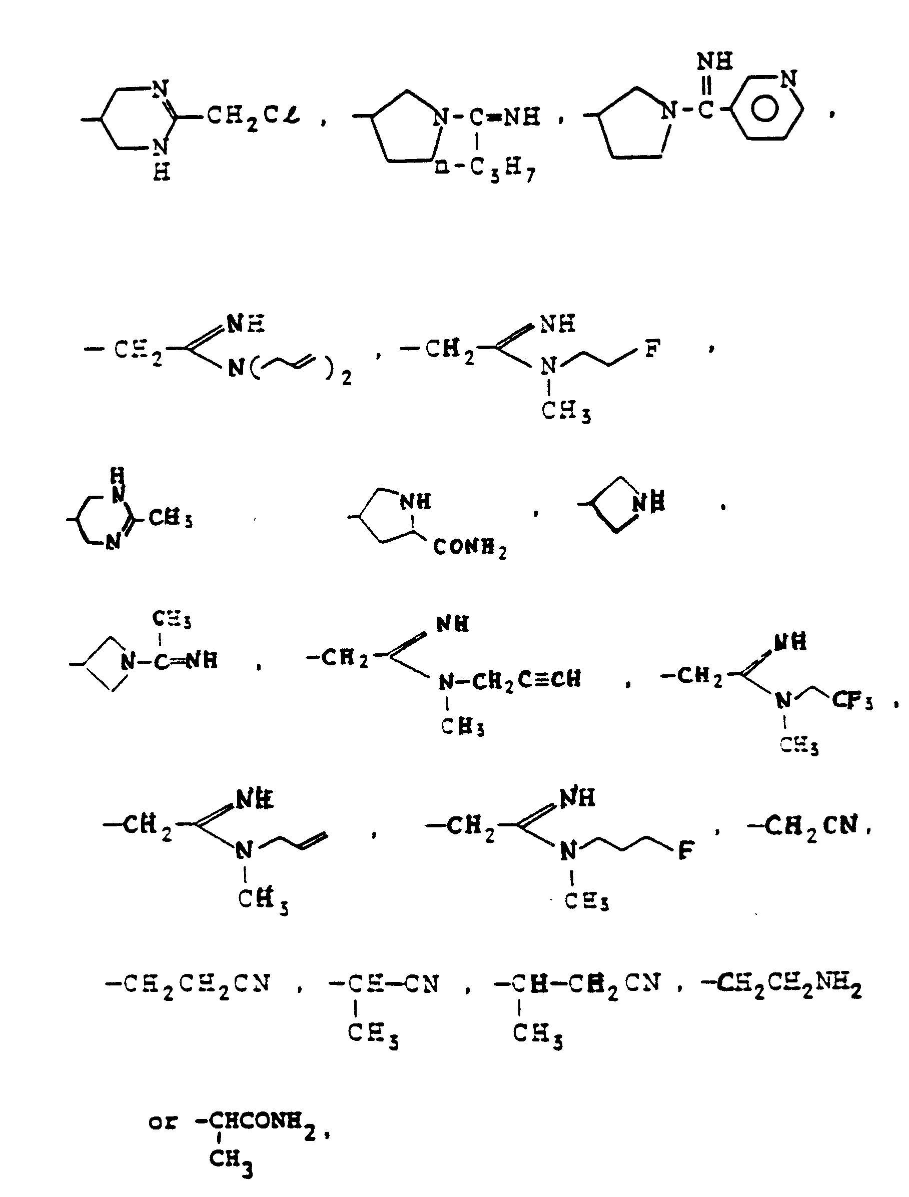 or a pharmaceutically acceptable salt thereof.

10. A method as claimed in Claim 9, wherein said antibiotic is:
(5R,6S)-2-{2-[(aminomethylene)amino]ethylthio}-6-[1(R)-hydroxyethyl]-2-carbapenem-3-carboxylic acid
(5R,6S)-2-[(3S)-1-acetimidoylpyrrolidin-3-ylthio]-6-[1(R)-hydroxyethyl]-2-carbapenem-3-carboxylic acid
(5R,6S)-2-[(3R)-1-acetimidoylpyrrolidin-3-ylthio]-6-[1(R)-hydroxyethyl]-2-carbapenem-3-carboxylic acid
(5R,6S)-2-[(3R)-1-acetimidoylpyrrolidin-3-ylthio]-6-[1(R)-hydroxyethyl]-1(S)-methyl-2-carbapenem-3-carboxylic acid
(5R,6S)-2-[(3S)-1-acetimidoylpyrrolidin-3-ylthio]-6-[1(R)-hydroxyethyl]-1(R)-methyl-2-carbapenem-3-carboxylic acid
(5R,6S)-2-[(3S)-1-acetimidoylpyrrolidin-3-ylthio]-6-[1(R)-hydroxyethyl]-1(S)-methyl-2-carbapenem-3-carboxylic acid
or
(5R,6S)-2-[(3S)-1-acetimidoyl-5(S)-carbamoylpyrrolidin-3-ylthio]-6-[1(R)-hydroxyethyl]-2-carbapenem-3-carboxylic acid

11. A method as claimed in any one of Claims 1 to 10, wherein the weight ratio of said N-acylated amino acid to said antibiotic is from 0.1:1 to 4:1.

12. The use of:
(a) a penem antibiotic or a carbapenem antibiotic; and
(b) a pharmaceutically acceptable N-acylated derivative of an amino acid, the amino acid being ornithine, lysine, phenylglycine and phenylalanine, wherein the acyl group and amount are as defined in claim 1, for the manufacture of a one-part or two-part medicament for the treatment of bacterial infections.

13. The use as claimed in Claim 12, wherein said N-acylated amino acid is as defined in any one of Claims 2 to 8.

14. The use as claimed in Claim 12 or Claim 13, wherein said antibiotic is as defined in any one of Claims 9 and 10.

15. The use as claimed in any one of Claims 12 to 14, wherein the weight ratio of said N-acylated amino acid to said antibiotic is from 0.1:1 to 4:1.

## Revendications (Revendications pour l'(les) Etat(s) contractant(s) suivant(s): BE, CH, DE, FR, GB, IT, LI, NL, SE)

1. Composition comprenant : (a) un antibiotique qui est un antibiotique pénème ou un antibiotique carbapénème ; et (b) un amino-acide N-acylé pharmaceutiquement acceptable, lequel amino-acide est l'ornithine, la lysine, la phénylglycine ou la phénylalanine, et le groupe acyle est un groupe alcanoyle en C₁-c₁₈, un groupe alcénoyle en C₃-C₈, un groupe alcynoyle en C₃-C₈, un groupe acyle aromatique dont la partie aryle est un groupe aryle carbocyclique en C₆-C₁₄ et est non substitué ou a de 1 à 5 substituants alkyle en C₁-C₄, hydroxy, alcoxy en C₁-C₄, amino ou sulfonyle, un groupe cycloalcanecarbonyle dont lu partie cycloalcane est en C₃-C₈, un groupe alcoxycarbonyle en C₂-C₇ ou un groupe aralkyloxycarbonyle dont la partie aralkyle a de 7 à 9 atomes de carbone et est non substituée ou a de 1 à 5 substituants amino, alkyle en C₁-C₄, alcoxy en C₁-C₄ ou hydroxy ; ou un sel thérapeutiquement acceptable de celui-ci ; et où lu quantité dudit amino-acide N-acylé est suffisante pour atténuer toute toxicité rénale dudit antibiotique, de préférence dans un rapport pondéral de 0,1/1 à 4/1.

2. Composition selon la revendication 1, dans laquelle ledit amino-acide N-acylé est un composé de formule générale II :
R²-NH-(CH₂)ₙ-CH(COOH)-NHR³ (II)
dans laquelle :
n vaut 3 ou 4 ; et
R² et R³ sont semblables ou différents et représentent chacun un atome d'hydrogène ou un groupe acyle carboxylique, sous réserve que R² et R³ ne soient pas tous deux des atomes d'hydrogène ; dans laquelle le groupe acyle carboxylique est un groupe alcanoyle en C₁-C₁₈, un groupe alcénoyle en C₃-C₈, un groupe alcynoyle en C₃-C₈, un groupe acyle aromatique dont la partie aryle est un groupe aryle carbocyclique en C₆-C₁₄ qui est non substitué ou a de 1 à 5 substituants alkyle en C₁-C₄, hydroxy, alcoxy en C₁-C₄, amino ou sulfonyle, un groupe cycloalcanecarbonyle dont la partie cycloalcane est en C₃-C₈, un groupe alcoxycarbonyle en C₂-C₇ ou un groupe aralkyloxycarbonyle dont la partie aralkyle a de 7 à 9 atomes de carbone et est non substituée ou a de 1 à 5 substituants amino, alkyle en C₁-C₄, alcoxy en C₁-C₄ ou hydroxy ;
ou un sel pharmaceutiquement acceptable de celui-ci.

3. Composition selon la revendication 2, dans laquelle R² et R³ sont semblables ou différents et représentent chacun un atome d'hydrogène ou un groupe acétyle, benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluoyle, benzyloxycarbonyle ou 4-méthoxybenzyloxycarbonyle, sous réserve que R² et R³ ne soient pas tous deux des atomes d'hydrogène.

4. Composition selon la revendication 2, dans laquelle au moins un de R² et R³ représente un groupe acétyle et/ou benzoyle.

5. Composition selon la revendication 1, dans laquelle ledit amino-acide N-acylé est un composé de formule générale III :
Ph-(CH₂)ₘ-CH(COOH)-NHR⁴ (III)
dans laquelle :
Ph représente un groupe phényle ;
m vaut 0 ou 1 ; et
R⁴ représente un groupe acyle carboxylique tel que défini ;
ou un sel pharmaceutiquement acceptable de celui-ci.

6. Composition selon la revendication 5, dans laquelle R⁴ représente un groupe acétyle, benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluoyle, benzyloxycarbonyle ou 4-méthoxybenzyloxycarbonyle.

7. Composition selon la revendication 5, dans laquelle R⁴ représente un groupe acétyle ou benzoyle.

8. Composition selon la revendication 1, dans laquelle ledit amino-acide N-acylé est :
la N^{α},N^{δ}-dibenzoylornithine, la N^{α},N^{δ}-dicyclohexanecarbonylornithine, la N^{α}-benzoyl-N^{δ}-cyclohexanecarbonylornithine, la N^{α}-benzoyl-N^{δ}-acétylornithine, la N^{α},N^{ε}-dibenzoyllysine, la N^{α},N^{ε}-dicyclohexanecarbonyllysine, la N^{α}-benzoyl-N^{ε}-cyclohexanecarbonyllysine, la N^{α}-benzoyl-N^{ε}-acétyllysine, la N-benzoylphénylglycine, la N-benzoylphénylalanine, la N-cyclohexanecarbonylphénylglycine, la N-cyclohexanecarbonylphénylalanine, la N^{α}-cyclohexanecarbonyl-N^{ε}-acétyllysine ou la N^{α}-cyclohexanecarbonyl-N^{δ}-acétylornithine.

9. Composition selon l'une quelconque des revendications 1 à 8, dans laquelle ledit antibiotique est un composé de formule générale I : 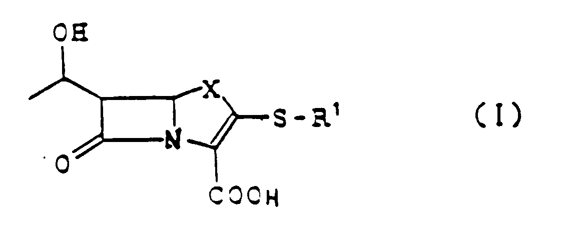 dans laquelle :
X représente un atome de soufre, un groupe méthylène ou un groupe méthylène ayant 1 ou 2 substituants méthyle ;
et
R¹ représente un groupe de formule : 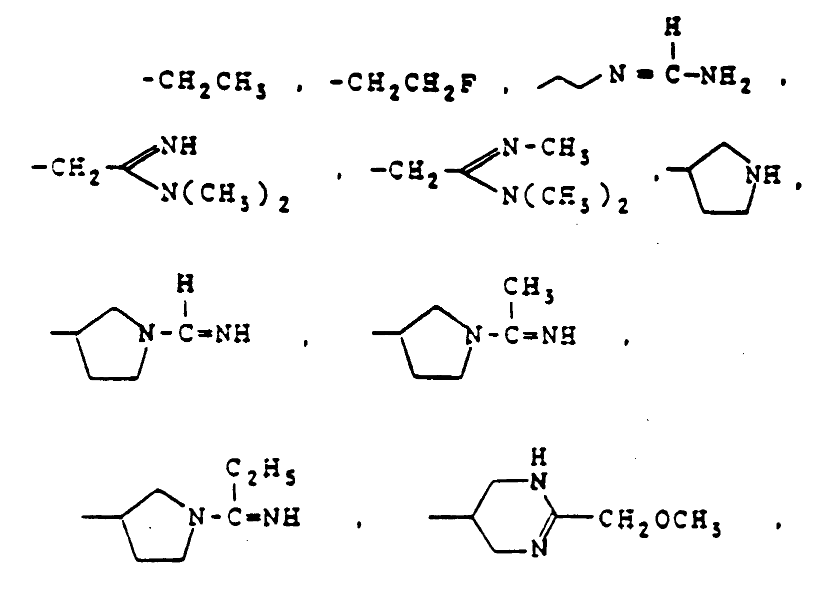 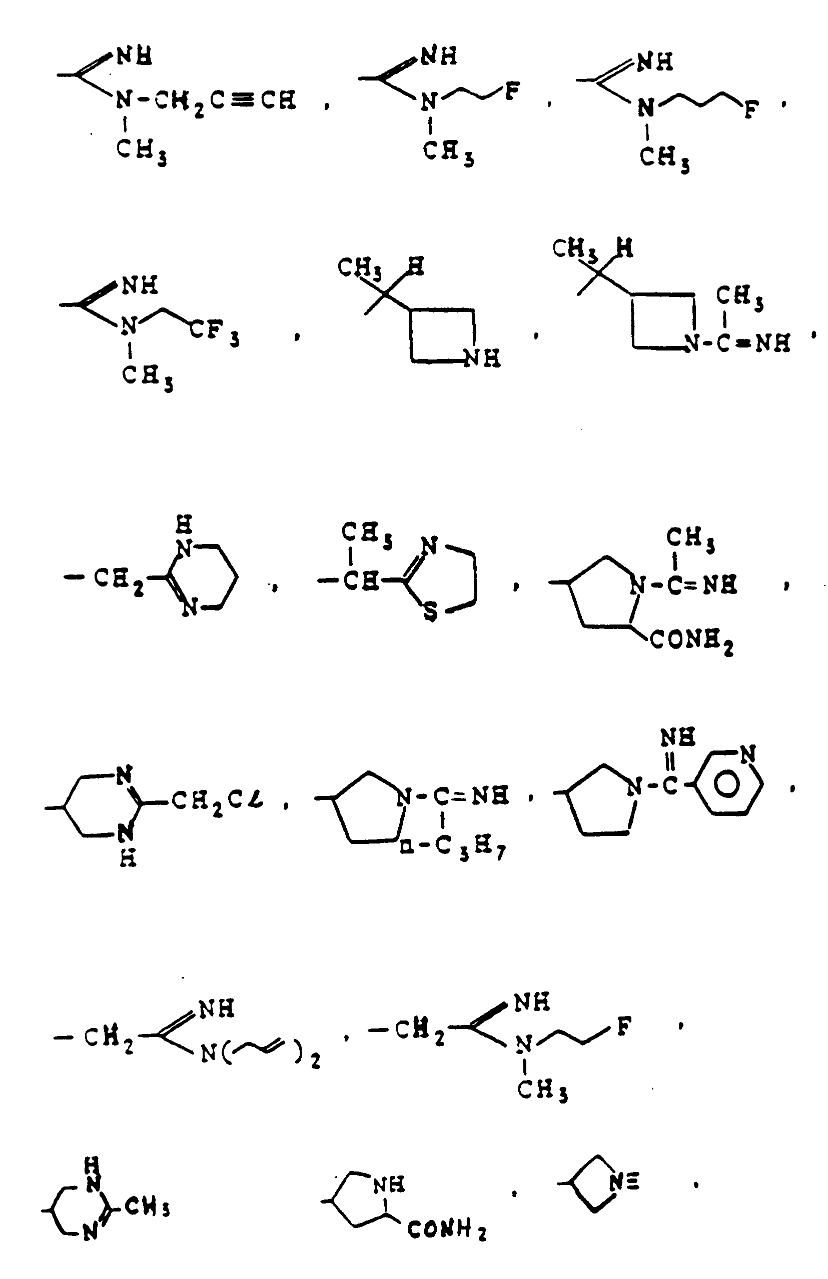 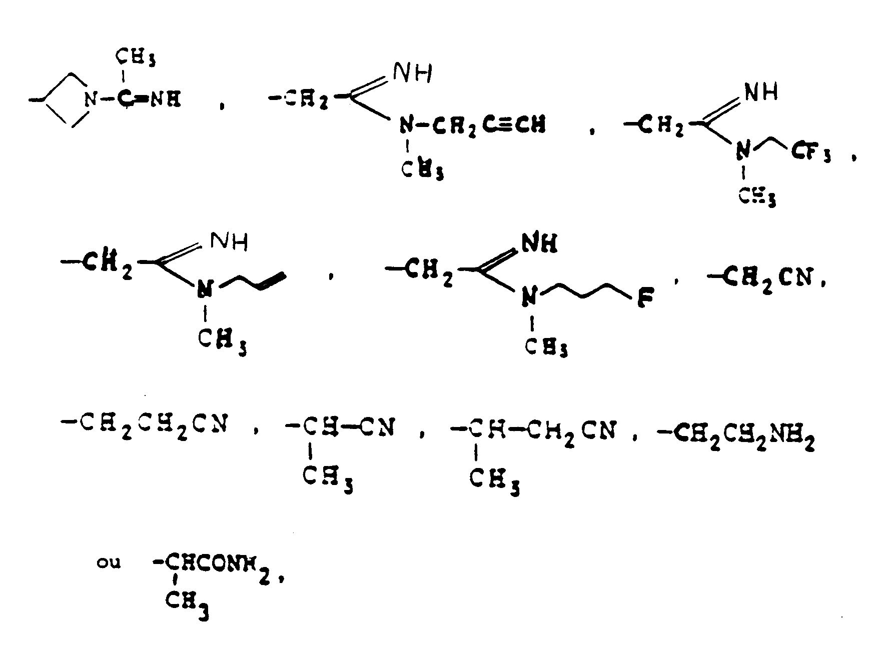 ou un sel pharmaceutiquement acceptable de celui-ci.

10. Composition selon la revendication 9, dans laquelle ledit antibiotique est :
l'acide (5R,6S)-2-{2-[(aminométhylène)amino]éthylthio}-6-[1(R)-hydroxyéthyl]-2-carbapénème-3-carboxylique,
l'acide (5R,6S)-2-[(3S)-1-acétimidoylpyrrolidine-3-ylthio]-6-[1(R)-hydroxyéthyl]-2-carbapénème-3-carboxylique,
l'acide (5R,6S)-2-[(3R)-1-acétimidoylpyrrolidine-3-ylthio]-6-[1(R)-hydroxyéthyl]-2-carbapénème-3-carboxylique,
l'acide (5R,6S)-2-[(3R)-1-acétimidoylpyrrolidine-3-ylthio]-6-[1(R)-hydroxyéthyl]-1-(S)-méthyl-2-carbapénème-3-carboxylique,
l'acide (5R,6S)-2-[(3S)-1-acétimidoylpyrrolidine-3-ylthio]-6-[1(R)-hydroxyéthyl]-1(R)-méthyl-2-carbapénème-3-carboxylique,
l'acide (5R,6S)-2-[(3S)-1-acétimidoylpyrrolidine-3-ylthio]-6-[1(R)-hydroxyéthyl]-1(S)-méthyl-2-carbapénème-3-carboxylique, ou
l'acide (5R,6S)-2-[(3S)-1-acétimidoyl-5(S)-carbamoylpyrrolidine-3-ylthio]-6-[1(R)-hydroxyéthyl]-2-carbapénème-3-carboxylique.

11. Composition selon l'une quelconque des revendications 1 à 10, dans laquelle le rapport pondéral dudit amino-acide N-acylé audit antibiotique est de 0,1/1 à 4/1.

12. Utilisation de :
(a) un antibiotique pénème ou un antibiotique carbapénème ; et
(b) un dérivé N-acylé pharmaceutiquement acceptable d'un amino-acide, l'amino-acide étant l'ornithine, la lysine, la phénylglycine et la phénylalanine, le groupe acyle et la quantité étant tels que définis dans la revendication 1, pour la fabrication d'un médicament en une seule partie ou en deux parties pour le traitement des infections bactériennes.

13. Utilisation selon la revendication 12, dans laquelle ledit amino-acide N-acylé est tel que défini dans l'une quelconque des revendications 2 à 8.

14. Utilisation selon la revendication 12 ou la revendication 13, dans laquelle ledit antibiotique est tel que défini dans l'une quelconque des revendications 9 et 10.

15. Utilisation selon l'une quelconque des revendications 12 à 14, dans laquelle le rapport pondéral dudit amino-acide N-acylé audit antibiotique est de 0,1/1 à 4/1.

16. Préparation pharmaceutique conditionnée, pour le traitement d'un mammifère souffrant d'une infection bactérienne, comprenant :
(a) dans une partie, un antibiotique pénème ou un antibiotique carbapénème ; et
(b) dans une autre partie, un dérivé N-acylé pharmaceutiquement acceptable d'un amino-acide, l'amino-acide étant l'ornithine, la lysine, la phénylglycine ou la phénylalanine, et le groupe acyle et la quantité étant tels que définis dans la revendication 1.

17. Préparation selon la revendication 16, dans laquelle ledit amino-acide N-acylé est tel que défini dans l'une quelconque des revendications 2 à 10.

18. Préparation selon la revendication 16 ou la revendication 17, dans laquelle ledit antibiotique est tel que défini dans l'une quelconque des revendications 9 et 10.

19. Préparation selon l'une quelconque des revendications 16 à 18, dans laquelle le rapport pondéral dudit amino-acide N-acylé audit antibiotique est de 0,1/1 à 4/1.

## Revendications (Revendications pour l'(les) Etat(s) contractant(s) suivant(s): AT)

1. Procédé pour préparer une composition pharmaceutique par mélange de : (a) un antibiotique qui est un antibiotique pénème ou un antibiotique carbapénème ; et (b) un amino-acide N-acylé pharmaceutiquement acceptable, lequel amino-acide est l'ornithine, la lysine, la phénylglycine ou la phénylalanine, et le groupe acyle est un groupe alcanoyle en C₁-C₁₈, un groupe alcénoyle en C₃-C₈, un groupe alcynoyle en C₃-C₈, un groupe acyle aromatique dont la partie aryle est un groupe aryle carbocyclique en C₆-C₁₄ et est non substitué ou a de 1 à 5 substituants alkyle en C₁-C₄, hydroxy, alcoxy en C₁-C₄, amino ou sulfonyle, un groupe cycloalcanecarbonyle dont la partie cycloalcane est en C₃-C₈, un groupe alcoxycarbonyle en C₂-C₇ ou un groupe aralkyloxycarbonyle dont la partie aralkyle a de 7 à 9 atomes de carbone et est non substituée ou a de 1 à 5 substituants amino, alkyle en C₁-C₄, alcoxy en C₁-C₄ ou hydroxy ; ou un sel pharmaceutiquement acceptable de celui-ci ; et où la quantité dudit amino-acide N-acylé est suffisante pour atténuer toute toxicité rénale dudit antibiotique, de préférence dans un rapport pondéral de 0,1/1 à 4/1.

2. Procédé selon la revendication 1, dans lequel ledit amino-acide N-acylé est un composé de formule générale II :
R²-NH-(CH₂)ₙ-CH(COOH)-NHR³ (II)
dans laquelle :
n vaut 3 ou 4 ; et
R² et R³ sont semblables ou différents et représentent chacun un atome d'hydrogène ou un groupe acyle carboxylique, sous réserve que R² et R³ ne soient pas tous deux des atomes d'hydrogène ; dans laquelle le groupe acyle carboxylique est un groupe alcanoyle en C₁-C₁₈, un groupe alcénoyle en C₃-C₈, un groupe alcynoyle en C₃-C₈, un groupe acyle aromatique dont la partie aryle est un groupe aryle carbocyclique en C₆-C₁₄ qui est non substitué ou a de 1 à 5 substituants alkyle en C₁-C₄, hydroxy, alcoxy en C₁-C₄, amino ou sulfonyle, un groupe cycloalcanecarbonyle dont la partie cycloalcane est en C₃-C₈, un groupe alcoxycarbonyle en C₂-C₇ ou un groupe aralkyloxycarbonyle dont la partie aralkyle a de 7 à 9 atomes de carbone et est non substituée ou a de 1 à 5 substituants amino, alkyle en C₁-C₄, alcoxy en C₁-C₄ ou hydroxy ;
ou un sel pharmaceutiquement acceptable de celui-ci.

3. Procédé selon la revendication 2, dans lequel R² et R³ sont semblables ou différents et représentent chacun un atome d'hydrogène ou un groupe acétyle, benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluoyle, benzyloxycarbonyle ou 4-méthoxybenzyloxycarbonyle, sous réserve que R² et R³ ne soient pas tous deux des atomes d'hydrogène.

4. Procédé selon la revendication 2, dans lequel au moins un de R² et R³ représente un groupe acétyle et/ou benzoyle.

5. Procédé selon la revendication 1, dans lequel ledit amino-acide N-acylé est un composé de formule générale III :
Ph-(CH₂)ₘ-CH(COOH)-NHR⁴ (III)
dans laquelle :
Ph représente un groupe phényle ;
m vaut 0 ou 1 ; et
R⁴ représente un groupe acyle carboxylique tel que défini ;
ou un sel pharmaceutiquement acceptable de celui-ci.

6. Procédé selon la revendication 5, dans lequel R⁴ représente un groupe acétyle, benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluoyle, benzyloxycarbonyle ou 4-méthoxybenzyloxycarbonyle.

7. Procédé selon la revendication 5, dans lequel R⁴ représente un groupe acétyle ou benzoyle.

8. Procédé selon la revendication 1, dans lequel ledit amino-acide N-acylé est :
la N^{α},N^{δ}-dibenzoylornithine, la N^{α},N^{δ}-dicyclohexanecarbonylornithine, la N^{α}-benzoyl-N^{δ}-cyclohexanecarbonylornithine, la N^{α}-benzoyl-N^{δ}-acétylornithine, la N^{α},N^{ε}-dibenzoyllysine, la N^{α},N^{ε}-dicyclohexanecarbonyllysine, la N^{α}-benzoyl-N^{ε}-cyclohexanecarbonyllysine, la N^{α}-benzoyl-N^{ε}-acétyllysine, la N-benzoylphénylglycine, la N-benzoylphénylalanine, la N-cyclohexanecarbonylphénylglycine, la N-cyclohexanecarbonylphénylalanine, la N^{α}-cyclohexanecarbonyl-N^{ε}-acétyllysine ou la N^{α}-cyclohexanecarbonyl-N^{δ}-acétylornithine.

9. Procédé selon l'une quelconque des revendications 1 à 8, dans lequel ledit antibiotique est un composé de formule générale I : 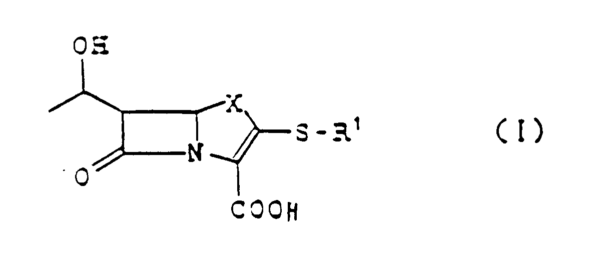 dans laquelle :
X représente un atome de soufre, un groupe méthylène ou un groupe méthylène ayant 1 ou 2 substituants méthyle ;
et
R¹ représente un groupe de formule : 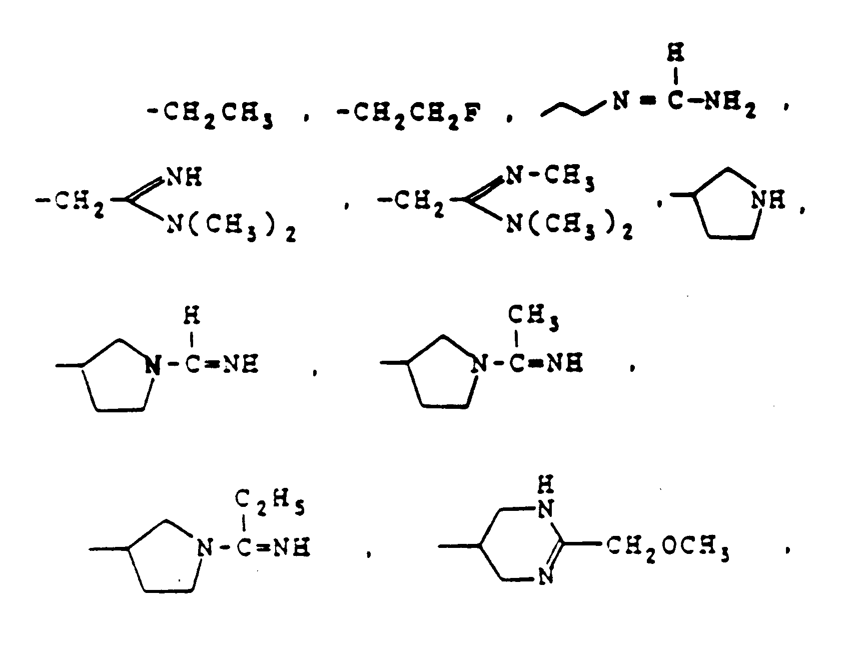 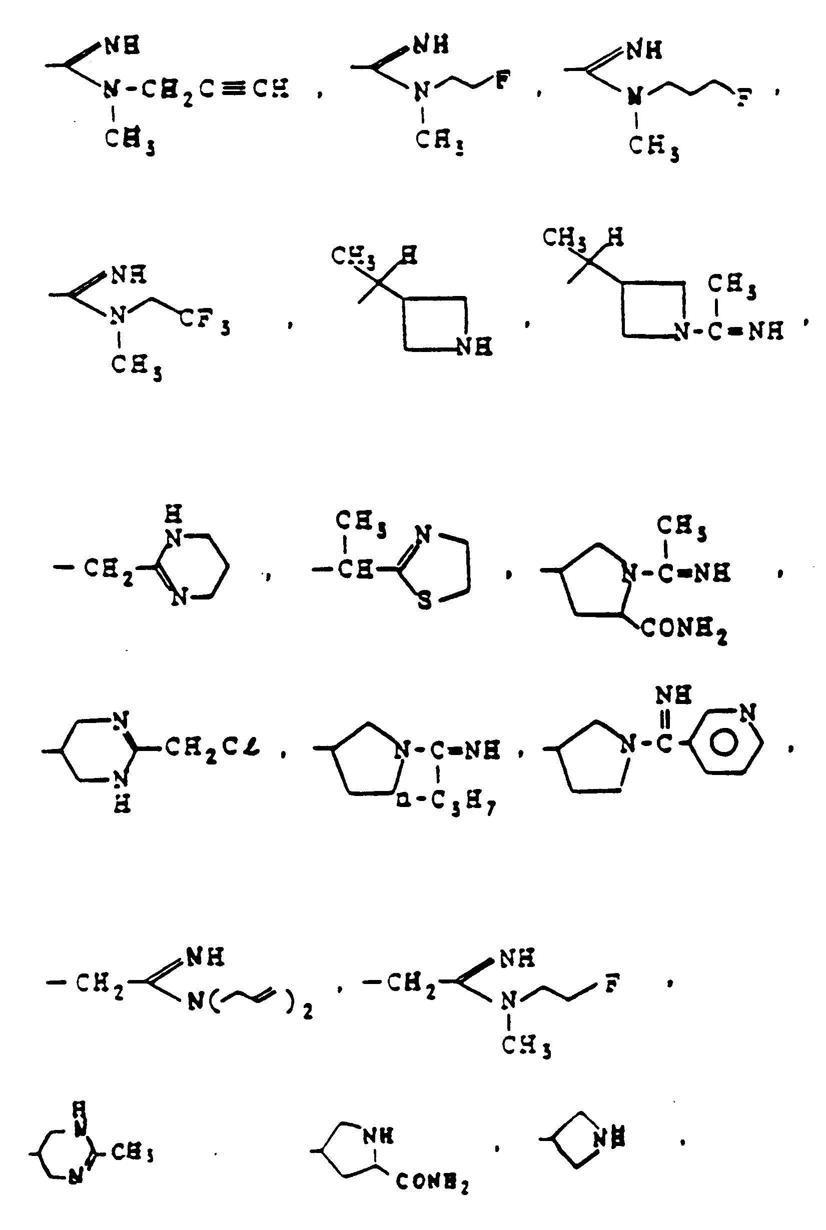 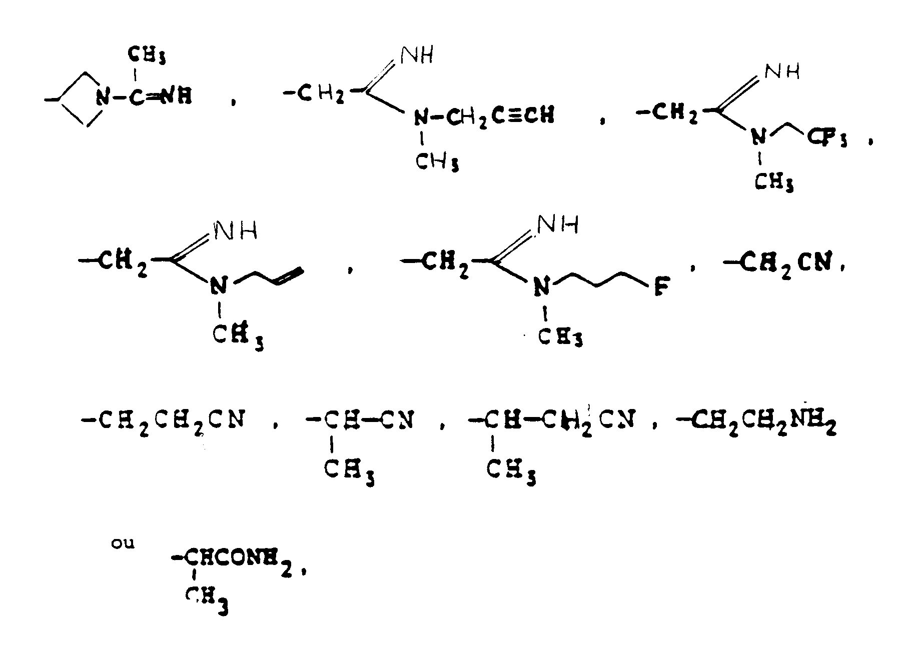 ou un sel pharmaceutiquement acceptable de celui-ci.

10. Procédé selon la revendication 9, dans lequel ledit antibiotique est :
l'acide (5R,6S)-2-{2-[(aminométhylène)amino]éthylthio}-6-[1(R)-hydroxyéthyl]-2-carbapénème-3-carboxylique,
l'acide (5R,6S)-2-[(3S)-1-acétimidoylpyrrolidine-3-ylthio]-6-[1(R)-hydroxyéthyl]-2-carbapénème-3-carboxylique,
l'acide (5R,6S)-2-[(3R)-1-acétimidoylpyrrolidine-3-ylthio]-6-[1(R)-hydroxyéthyl]-2-carbapénème-3-carboxylique,
l'acide (5R,6S)-2-[(3R)-1-acétimidoylpyrrolidine-3-ylthio]-6-[1(R)-hydroxyéthyl]-1(S)-méthyl-2-carbapénème-3-carboxylique,
l'acide (5R,6S)-2-[(3S)-1-acétimidoylpyrrolidine-3-ylthio]-6-[1(R)-hydroxyéthyl]-1(R)-méthyl-2-carbapénème-3-carboxylique,
l'acide (5R,6S)-2-[(3S)-1-acétimidoylpyrrolidine-3-ylthio]-6-[1(R)-hydroxyéthyl]-1-(S)-méthyl-2-carbapénème-3-carboxylique, ou
l'acide (5R,6S)-2-[(3S)-1-acétimidoyl-5(S)-carbamoylpyrrolidine-3-ylthio]-6-[1(R)-hydroxyéthyl]-2-carbapénème-3-carboxylique.

11. Procédé selon l'une quelconque des revendications 1 à 10, dans lequel le rapport pondéral dudit amino-acide N-acylé audit antibiotique est de 0,1/1 à 4/1.

12. Utilisation de :
(a) un antibiotique pénème ou un antibiotique carbapénème ; et
(b) un dérivé N-acylé pharmaceutiquement acceptable d'un amino-acide, l'amino-acide étant l'ornithine, la lysine, la phénylglycine et la phénylalanine, le groupe acyle et la quantité étant tels que définis dans la revendication 1, pour la fabrication d'un médicament en une seule partie ou en deux parties pour le traitement des infections bactériennes.

13. Utilisation selon la revendication 12, dans laquelle ledit amino-acide N-acylé est tel que défini dans l'une quelconque des revendications 2 à 8.

14. Utilisation selon la revendication 12 ou la revendication 13, dans laquelle ledit antibiotique est tel que défini dans l'une quelconque des revendications 9 et 10.

15. Utilisation selon l'une quelconque des revendications 12 à 14, dans laquelle le rapport pondéral dudit amino-acide N-acylé audit antibiotique est de 0,1/1 à 4/1.

## Patentansprüche (Patentansprüche für folgende(n) Vertragsstaat(en): BE, CH, DE, FR, GB, IT, LI, NL, SE)

1. Eine Zusammensetzung, welch enthält: (a) ein Antibioticum, welches ein Penem-Antibioticum oder ein Carbapenem-Antibioticum ist; und (b) eine pharmazeutisch vertragliche N-acylierte Amino-Säure, in welcher die Amino-Säure Ornithin, Lysin, Phenylglycin oder Phenylalanin ist, und die Acyl-Gruppe eine C₁-C₁₈ Alkanoyl-Gruppe, eine C₃-C₈ Alkenoyl-Gruppe, eine C₃-C₈ Alkynoyl-Gruppe, eine aromatische Acyl-Gruppe, in welcher der Aryl-Teil C₆-C₁₄ carbocyclisches Aryl und unsubstituiert ist oder 1 bis 5 C₁-C₄ Alkyl-, Hydroxy-, C₁-C₄ Alkoxy-, Amino- oder Sulphonyl-Substituenten hat, eine Cycloalkancarbonyl-Gruppe, worin der Cycloalkan-Teil C₃-C₈, eine C₂-C₇ Alkoxycarbonyl-Gruppe oder eine Aralkyloxycarbonyl-Gruppe ist, worin der Aralkyl-Teil 7 bis 9 Kohlenstoffatome hat und unsubstituiert ist oder 1 bis 5 Amino-, C₁-C₄ Alkyl-, C₁-C₄Alkoxy- oder Hydroxy-Substituenten hat, ist; oder ein therapeutisch verträgliches Salz davon; und worin die Menge der erwähnten N-acylierten Amino-Säure ausreicht, um jede renale Toxizität des erwähnten Antibioticums zu mildern, vorzugsweise im Gewichtsverhältnis von 0,1:1 bis 4:1.

2. Eine Zusammensetzung nach Anspruch 1, worin die erwähnte N-acylierte Aminosäure eine Verbindung dar allgemeinen Formel II ist:
R²-NH-(CH₂)ₙ-CH(COOH)-NHR³ (II)
worin:
n 3 oder 4 ist; und
R² und R³ gleich oder verschieden sind und jedes ein Wasserstoffatom oder eine carbocyclische Acyl-Gruppe bedeutet, wobei jedoch R² und R³ nicht beide Wasserstoffatome sind; wobei die carbocyclische Acyl-Gruppe eine C₁-C₁₈ Alkanoyl-Gruppe, eine C₃-C₈ Alkenoyl-Gruppe, eine C₃-C₈ Alkynoyl-Gruppe, eine aromatische Acyl-Gruppe, in welcher der Aryl-Teil C₆-C₁₄ carbocyclisches Aryl und unsubstituiert ist oder 1 bis 5 C₁-C₄ Alkyl-, Hydroxy-, C₁-C₄ Alkoxy-, Amino- oder Sulphonyl-Substituenten hat, eine Cycloalkancarbonyl-Gruppe, in welcher der Cycloalkan-Teil C₃-C₈ ist,
eine C₂-C₇ Alkoxycarbonyl-Gruppe oder eine Aralkyloxycarbonyl-Gruppe ist, worin der Aralkyl-Teil 7 bis 9 Kohlenstoffatome hat und unsubstituiert ist oder 1 bis 5 Amino-, C₁-C₄ Alkyl-, C₁-C₄ Alkoxy- oder Hydroxy-Substituenten hat;
oder ein pharmazeutisch verträgliches Salz davon.

3. Eine Zusammensetzung nach Anspruch 2, worin R² und R³ gleich oder verschieden sind und jedes ein Wasserstoffatom oder eine Acetyl-, Benzoyl-, Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl-, oder 4-Methoxybenzyloxycarbonyl-Gruppe bedeutet, wobei jedoch R² und R³ nicht beide Wasserstoffatome sind.

4. Eine Zusammensetzung nach Anspruch 2, worin zumindest eines von R² und R³ eine Acetyl- und/oder Benzoyl-Gruppe bedeutet.

5. Eine Zusammensetzung nach Anspruch 1, worin die erwähnte N-acylierte Aminosäure eine Verbindung der allgemeinen Formel III ist:
Ph-(CH₂)ₘ-CH(COOH)-NHR⁴ (III)
worin:
Ph eine Phenyl-Gruppe bedeutet;
m 0 oder 1 ist; und
R⁴ eine carboxylische Acyl-Gruppe wie definiert bedeutet;
oder ein pharmazeutisch verträgliches Salz davon.

6. Eine Zusammensetzung nach Anspruch 5, worin R⁴ eine Acetyl, Benzoyl-, Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl- oder 4-Methoxybenzyloxycarbonyl-Gruppe bedeutet.

7. Eine Zusammensetzung nach Anspruch 5, worin R⁴ eine Acetyl- oder Benzoyl-Gruppe bedeutet.

8. Eine Zusammensetzung nach Anspruch 1, worin die erwähnte
N-acylierte Aminosäure
N^{α},N^{δ}-Dibenzoylornithin
N^{α},N^{δ}-Dicyclohexancarbonylornithin
N^{α}-Benzoyl-N^{δ}-Cyclohexancarbonylornithin
N^{α}-Benzoyl-N^{δ}-Acetylornithin
N^{α},N^{ε}-Dibenzoyllysin
N^{α},N^{ε}-Dicyclohexancarbonyllysin
N^{α}-Benzoyl-N^{ε}-Cyclohexancarbonyllysin
N^{α}-Benzoyl-N^{ε}-Acetyllysin
N-Benzoylphenylglycin
N-Benzoylphenylalanin
N-Cyclohexancarbonylphenylglycin
N-Cyclohexancarbonylphenylalanin
N^{α}-Cyclohexancarbonyl-N^{ε}-Acetyllysin
oder
N^{α}-Cyclohexancarbonyl-N^{δ}-Acetylornithin
ist.

9. Eine Zusammensetzung nach einem der Ansprüche 1 bis 8, worin das erwähnte Antibioticum eine Verbindung der allgemeinen Formel I ist: 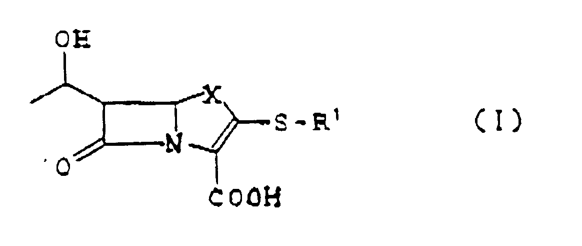 worin:
X ein Schwefelatom, eine Methylen-Gruppe oder eine Methylen-Gruppe mit 1 oder 2 Methyl-Substituenten bedeutet;
und
R¹ eine Gruppe der Formel 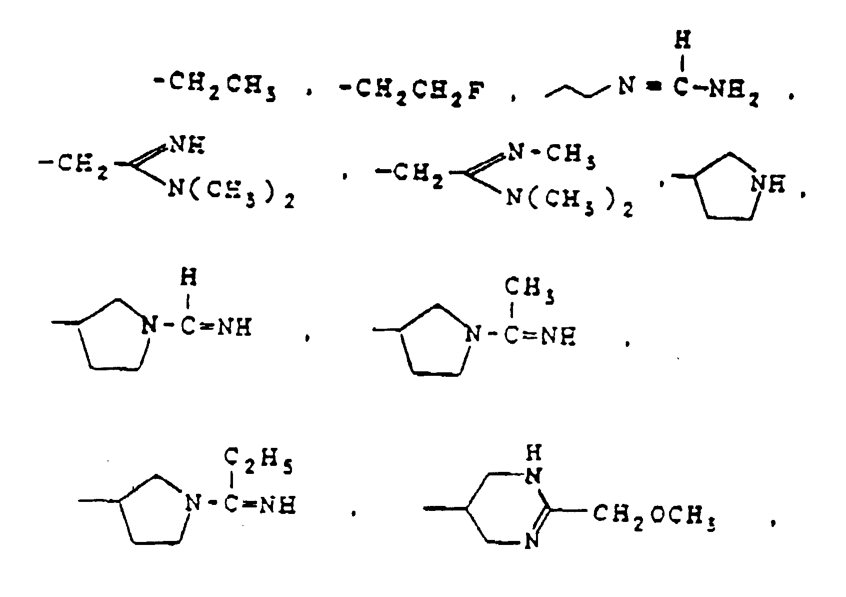 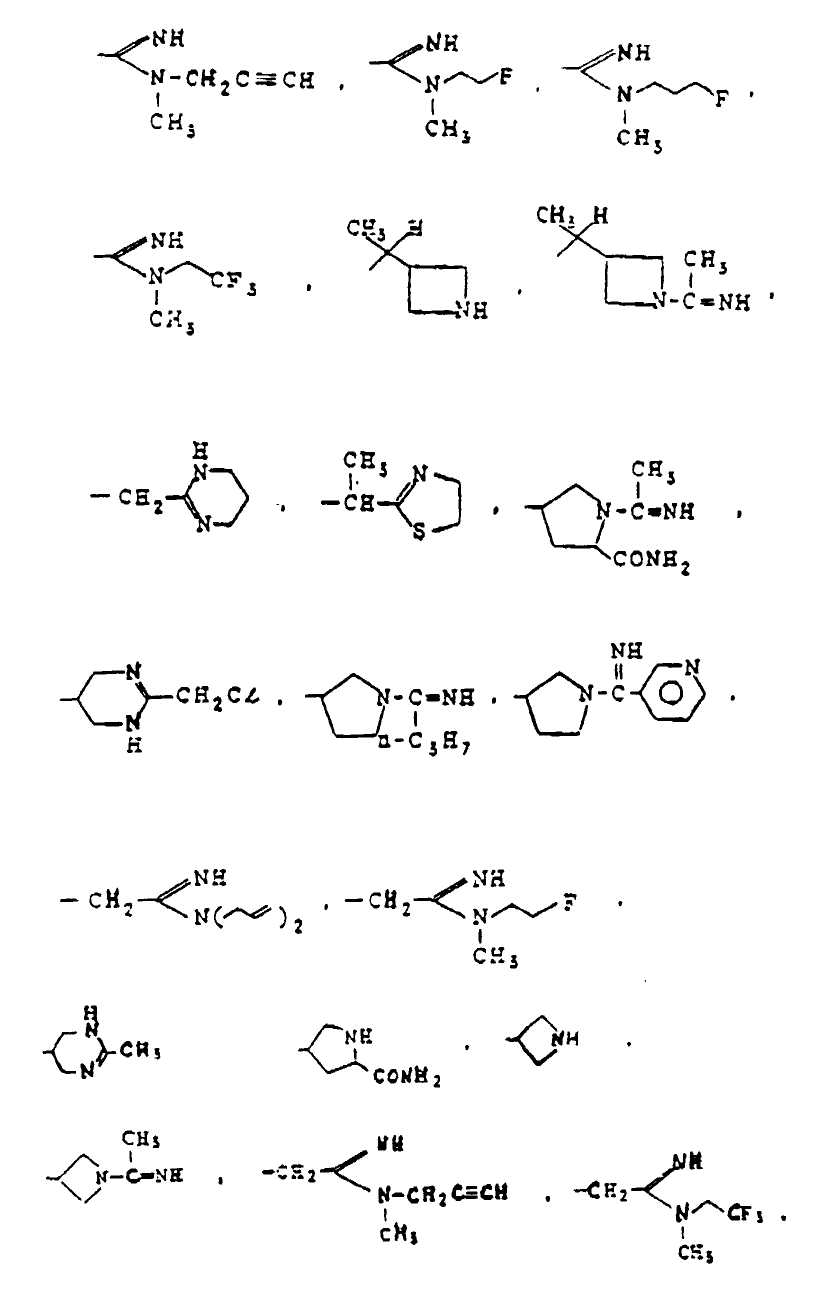 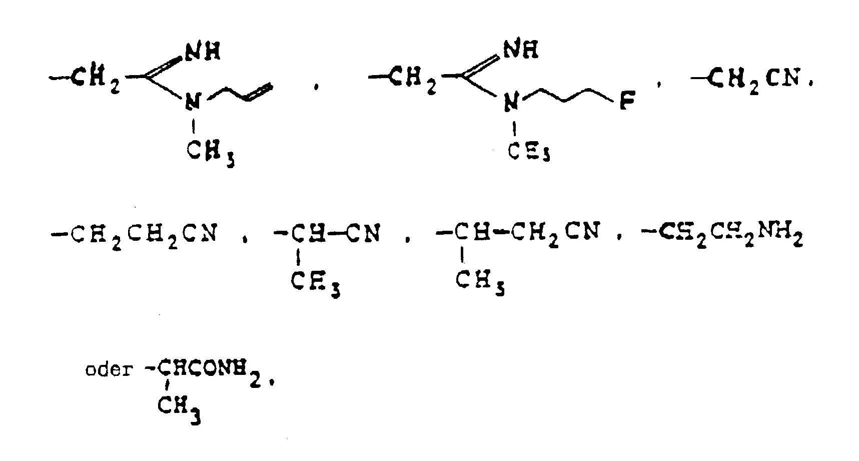 bedeutet
oder ein pharmazeutisch verträgliches Salz davon.

10. Eine Zusammensetzung nach Anspruch 9, worin das erwähnte Antibiotikum
(5R,6S)-2-{2- [(Aminomethylen)Amino]Äthylthio}-6-[1(R)Hydroxyäthyl]-2-Carbapenem-3-Carboxyl-Säure
(5R,6S)-2-[(3S)-1-Acetimidoylpyrrolidin-3-Ylthio]-6-[1(R)Hydroxyäthyl] -2-Carbapenem-3-Carboxyl-Säure
(5R,6S)-2-[(3R)-1-Acetimidoylpyrrolidin-3-Ylthio]-6-[1(R)-Hydroxyäthyl] -2-Carbapenem-3-Carboxyl-Säure
(5R),6S)-2-[(3R)-1-Acetimidoylpyrrolidin-3-Ylthio]-6-[1(R)-Hydroxyäthyl] -1(S)-Methyl-2-Carbapenem-3-Carboxyl-Säure
(5R,6S)-2-[(3S)-1-Acetimidoylpyrrolidin-3-Ylthio]-6-[1(R)-Hydroxyäthyl] -1(R)-Methyl-2-Carbapenem-3-Carboxyl-Säure
(5R,6S)-2-[(3S)-1-Acetimidoylpyrrolidin-3-Ylthio]-6-[1(R)-Hydroxyäthyl] -1(S)-Methyl-2-Carbapenem-3-Carboxyl-Säure
oder
(5R,6S)-2- [(3S)-1-Acetimodoyl-5(S)-Carbamoylpyrrolidin-3-Ylthio] -6-[1(R)-Hydroxyäthyl]-2-Carbapenem-3-Carboxyl-Säure
ist.

11. Eine Zusammensetzung nach einem der Ansprüche 1 bis 10, wobei das Gewichtsverhältnis zwischen der erwähnten N-acylierten Amino-Säure und dem erwähnten Antibiotikum 0,1:1 bis 4:1 beträgt.

12. Die Verwendung:
(a) Eines Penem-Antibiotikums oder eines Carbapenem-Antibiotikums; und
(b) eines pharmazeutisch verträglichen N-acylierten Derivates einer Aminosäure, wobei die Aminosäure Ornithin, Lysin, Phenylglycin oder Phenylalanin ist, worin die Acyl-Gruppe und die Menge wie in Anspruch 1 definiert sind, zur Herstellung eines ein-teiligen oder zwei-teiligen Arzneimittels zur Behandlung bakterieller Infektionen.

13. Die Verwendung nach Anspruch 12, worin die erwähnte N-acylierte Aminosäure wie in einem der Ansprüche 2 bis 8 definiert ist.

14. Die Verwendung nach Anspruch 12 oder Anspruch 13, worin das erwähnte Antibiotikum wie in einem der Ansprüche 9 und 10 definiert ist.

15. Die Verwendung nach einem der Ansprüche 12 bis 14, worin das Gewichtsverhältnis zwischen der erwähnten N-acylierten Aminosäure und dem erwähnten Antibiotikum 0,1:1 bis 4:1 beträgt.

16. Ein verpacktes pharmazeutisches Präparat zur Behandlung eines Mammaleidens infolge bakterieller Infektion, welches enthält:
(a) in einem Teil ein Penem-Antibiotikum oder ein Carbapenem-Antibiotikum; und
(b) in einem anderen Teil ein pharmazeutisch verträgliches N-acyliertes Derivat einer Aminosäure, wobei die Aminosäure Ornithin, Lysin, Phenylglycin oder Phenylalanin ist, und wobei die Acylgruppe und die Menge wie in Anspruch 1 definiert sind.

17. Ein Präparat nach Anspruch 16, worin die erwähnte N-acylierte Aminosäure wie in einem der Ansprüche 2 bis 10 definiert ist.

18. Ein Präparat nach Anspruch 16 oder Anspruch 17, worin das erwähnte Antibiotikum wie in einem der Ansprüche 9 und 10 definiert ist.

19. Ein Präparat nach einem der Ansprüche 16 bis 18, worin das Gewichtsverhältnis zwischen der erwähnten N-acylierten Aminosäure und dem erwähnten Antibiotikum 0,1:1 bis 4:1 beträgt.

## Patentansprüche (Patentansprüche für folgende(n) Vertragsstaat(en): AT)

1. Ein Verfahren zur Herstellung einer pharmazeutischen Zusammensetzung dadurch, daß man vermischt: (a) ein Antibioticum, welches ein Penem-Antibioticum oder ein Carbapenem-Antibioticum ist; und (b) eine pharmazeutisch verträgliche N-acylierte Amino-Säure, in welcher die Amino-Säure Ornithin, Lysin, Phenylglycin oder Phenylalanin ist, und die Acyl-Gruppe eine C₁-C₁₈ Alkanoyl-Gruppe, eine C₃-C₈ Alkenoyl-Gruppe, eine C₃-C₈ Alkynoyl-Gruppe, eine aromatische Acyl-Gruppe, in welcher der Aryl-Teil C₆-C₁₄ carbocyclisches Aryl und unsubstituiert ist oder 1 bis 5 C₁-C₄ Alkyl-, Hydroxy-, C₁-C₄ Alkoxy-, Amino- oder Sulphonyl-Substituenten hat, eine Cycloalkancarbonyl-Gruppe, worin der Cycloalkan-Teil C₃-C₈, eine C₂-C₇ Alkoxycarbonyl-Gruppe oder eine Aralkyloxycarbonyl-Gruppe ist, worin der Aralkyl-Teil 7 bis 9 Kohlenstoffatome hat und unsubstituiert ist oder 1 bis 5 Amino-, C₁-C₄ Alkyl-, C₁-C₄Alkoxy- oder Hydroxy-Substituenten hat, ist; oder ein therapeutisch verträgliches Salz davon; und worin die Menge der erwähnten N-acylierten Amino-Säure ausreicht, um jede renale Toxizität des erwähnten Antibioticums zu mildern, vorzugsweise im Gewichtsverhältnis von 0,1:1 bis 4:1.

2. Ein Verfahren nach Anspruch 1, worin die erwähnte N-acylierte Aminosäure eine Verbindung der allgemeinen Formel II ist:
R²-NH-(CH₂)ₙ-CH(COOH)-NHR³ (II)
worin:
n 3 oder 4 ist; und
R² und R³ gleich oder verschieden sind und jedes ein Wasserstoffatom oder eine carbocyclische Acyl-Gruppe bedeutet, wobei jedoch R²und R³ nicht beide Wasserstoffatome sind; wobei die carbocyclische Acyl-Gruppe eine C₁-C₁₈ Alkanoyl-Gruppe, eine C₃-C₈ Alkenoyl-Gruppe, eine C₃-C₈ Alkynoyl-Gruppe, eine aromatische Acyl-Gruppe, in welcher der Aryl-Teil C₆-C₁₄ carbocyclisches Aryl und unsubstituiert ist oder 1 bis 5 C₁-C₄ Alkyl-, Hydroxy-, C₁-C₄ Alkoxy-, Amino- oder Sulphonyl-Substituenten hat, eine Cycloalkancarbonyl-Gruppe, in welcher der Cycloalkan-Teil C₃-C₈ ist, eine C₂-C₇ Alkoxycarbonyl-Gruppe oder eine Aralkyloxycarbonyl-Gruppe ist, worin der Aralkyl-Teil 7 bis 9 Kohlenstoffatome hat und unsubstituiert ist oder 1 bis 5 Amino-, C₁-C₄ Alkyl-, C₁-C₄ Alkoxy- oder Hydroxy-Substituenten hat;
oder ein pharmazeutisch verträgliches Salz davon.

3. Ein Verfahren nach Anspruch 2, worin R² und R³ gleich oder verschieden sind und jedes ein Wasserstoffatom oder eine Acetyl-, Benzoyl-, Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl-, oder 4-Methoxybenzyloxycarbonyl-Gruppe bedeutet, wobei jedoch R² und R³ nicht beide Wasserstoffatome sind.

4. Ein Verfahren nach Anspruch 2, worin zumindest eines von R² und R³ eine Acetyl- und/oder Benzoyl-Gruppe bedeutet.

5. Ein Verfahren nach Anspruch 1, worin die erwähnte N-acylierte Aminosäure eine Verbindung der allgemeinen Formel III ist:
Ph-(CH₂)ₘ-CH(COOH)-NHR⁴ (III)
worin:
Ph eine Phenyl-Gruppe bedeutet;
m 0 oder 1 ist; und
R⁴ eine carboxylische Acyl-Gruppe wie definiert bedeutet;
oder ein pharmazeutisch verträgliches Salz davon.

6. Ein Verfahren nach Anspruch 5, worin R⁴ eine Acetyl-, Benzoyl-, Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl- oder 4-Methoxybenzyloxycarbonyl-Gruppe bedeutet.

7. Ein Verfahren nach Anspruch 5, worin R⁴ eine Acetyl- oder Benzoyl-Gruppe bedeutet.

8. Ein Verfahren nach Anspruch 1, worin die erwähnte
N-acylierte Aminosäure
N^{α},N^{δ}-Dibenzoylornithin
N^{α},N^{δ}-Dicyclohexancarbonylornithin
N^{α}-Benzoyl-N^{δ}-Cyclohexancarbonylornithin
N^{α}-Benzoyl-N^{δ}-Acetylornithin
N^{α},N^{ε}-Dibenzoyllysin
N^{α},N^{ε}-Dicyclohexancarbonyllysin
N^{α}-Benzoyl-N^{ε}-Cyclohexancarbonyllysin
N^{α}-Benzoyl-N^{ε}-Acetyllysin
N-Benzoylphenylglycin
N-Benzoylphenylalanin
N-Cyclohexancarbonylphenylglycin
N-Cyclohexancarbonylphenylalanin
N^{α}-Cyclohexancarbonyl-N^{ε}-Acetyllysin
oder
N^{α}-Cyclohexancarbonyl-N^{δ}-Acetylornithin
ist.

9. Ein Verfahren nach einem der Ansprüche 1 bis 8, worin das erwähnte Antibioticum eine Verbindung der allgemeinen Formel I ist: 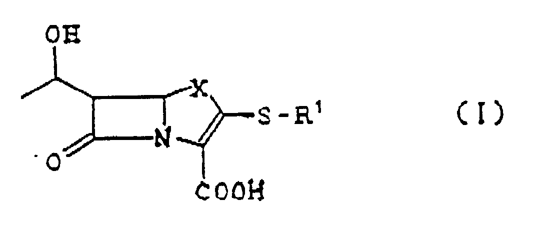 worin:
X ein Schwefelatom, eine Methylen-Gruppe oder eine Methylen-Gruppe mit 1 oder 2 Methyl-Substituenten bedeutet;
und
R¹ eine Gruppe der Formel 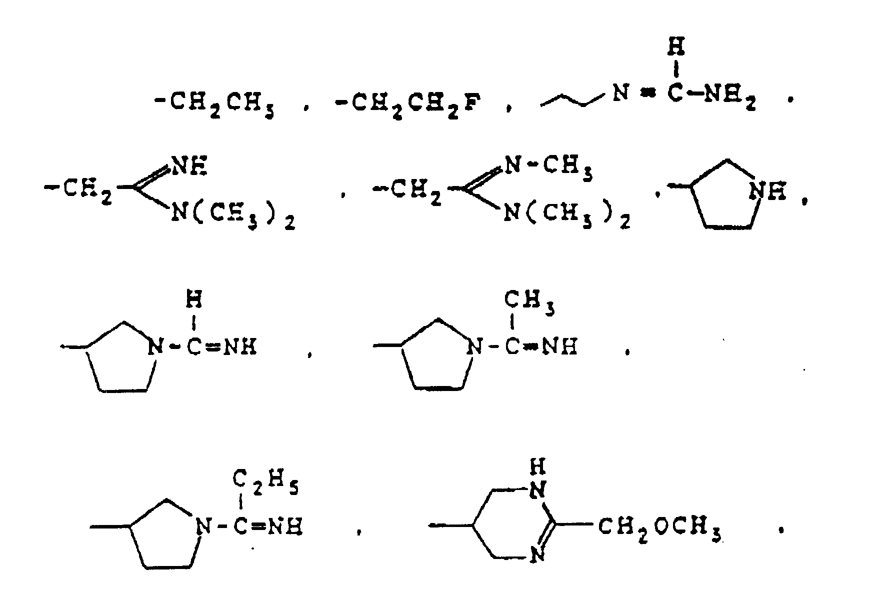 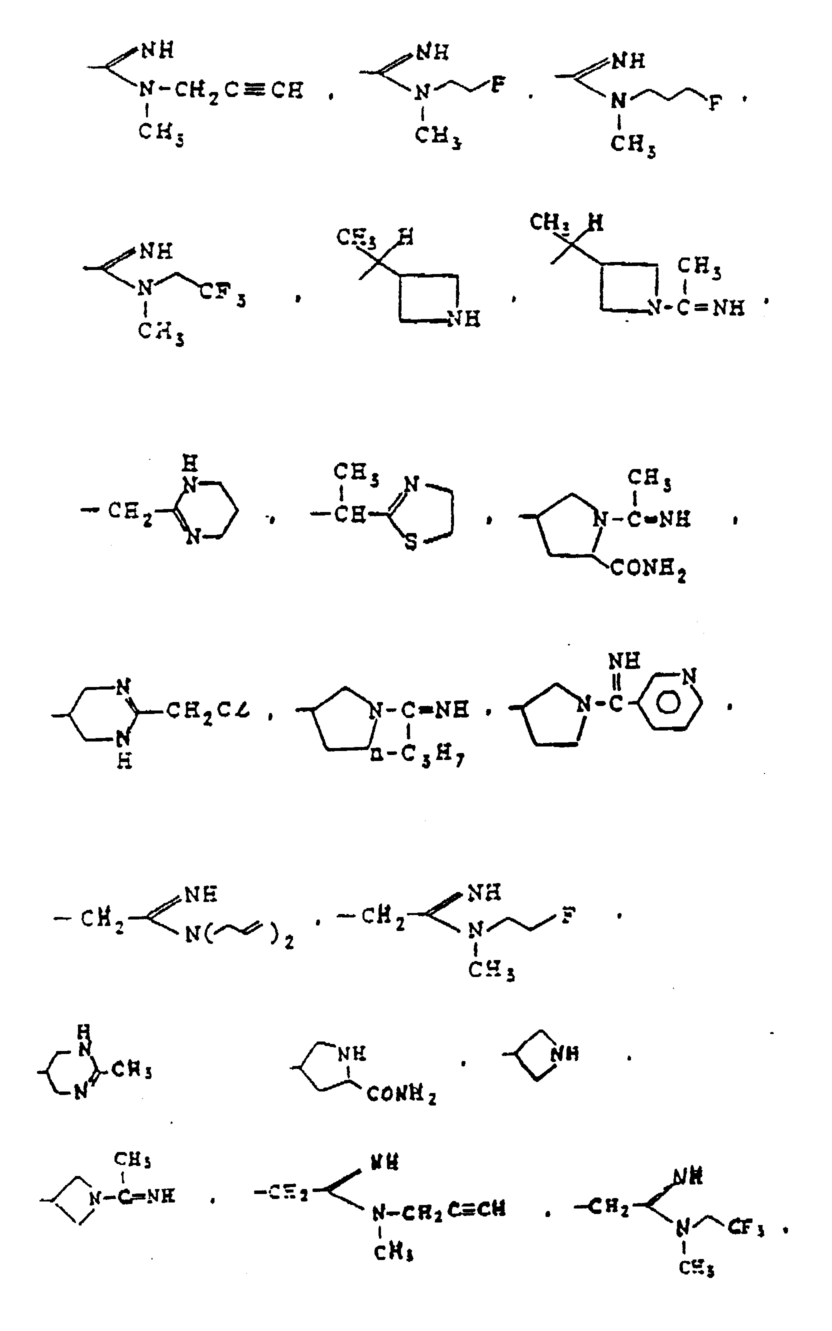 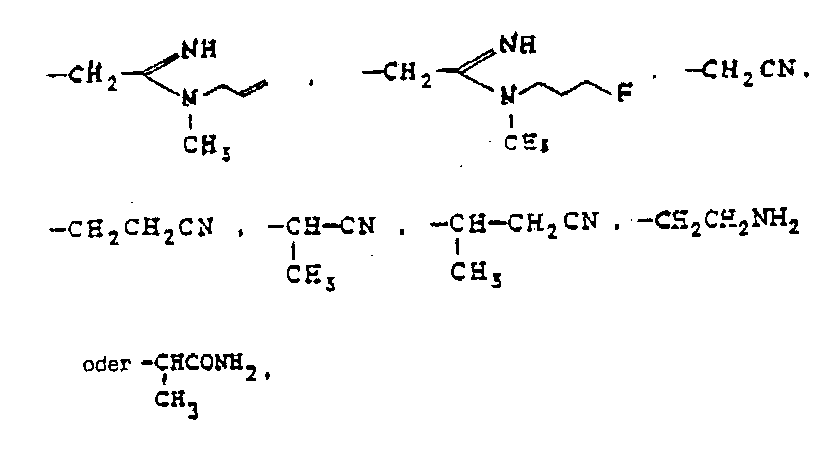 bedeutet
oder ein pharmazeutisch verträgliches Salz davon.

10. Ein Verfahren nach Anspruch 9, worin das erwähnte Antibiotikum
(5R,6S)-2-{2- [(Aminomethylen)Amino]Äthylthio}-6-[1(R)-Hydroxyäthyl]-2-Carbapenem-3-Carboxyl-Säure
(5R,6S)-2-[(3S)-1-Acetimidoylpyrrolidin-3-Ylthio]-6-[1(R)-Hydroxyäthyl] -2-Carbapenem-3-Carboxyl-Säure
(5R,6S)-2-[(3R)-1-Acetimidoylpyrrolidin-3-Ylthio]-6-[1(R)-Hydroxyäthyl] -2-Carbapenem-3-Carboxyl-Säure
(5R,6S)-2-[(3R)-1-Acetimidoylpyrrolidin-3-Ylthio]-6-[1(R)-Hydroxyäthyl] -1(S)-Methyl-2-Carbapenem-3-Carboxyl-Säure
(5R,6S)-2-[(3S)-1-Acetimidoylpyrrolidin-3-Ylthio]-6-[1(R)-Hydroxyäthyl] -1(R)-Methyl-2-Carbapenem-3-Carboxyl-Säure
(5R,6S)-2-[(3S)-1-Acetimidoylpyrrolidin-3-Ylthio]-6-[1(R)-Hydroxyäthyl] -1(S)-Methyl-2-Carbepenem-3-Carboxyl-Säure
oder
(5R,6S)-2- [(3S)-1-Acetimidoyl-5(S)-Carbomoylpyrrolidin-3-Ylthio] -6-[1(R)-Hydroxyäthyl]-2-Carbapenem-3-Carboxyl-Säure
ist.

11. Ein Verfahren nach einem der Ansprüche 1 bis 10, wobei das Gewichtsverhältnis zwischen der erwähnten N-acylierten Amino-Säure und dem erwähnten Antibiotikum 0,1:1 bis 4.1 beträgt.

12. Die Verwendung:
(a) Eines Penem-Antibiotikums oder eines Carbapenem-Antibiotikums; und
(b) eines pharmazeutisch verträglichen N-acylierten Derivates einer Aminosäure, wobei die Aminosäure Ornithin, Lysin, Phenylglycin oder Phenylalanin ist, worin die Acyl-Gruppe und die Menge wie in Anspruch 1 definiert sind, zur Herstellung eines ein-teiligen oder zwei-teiligen Arzneimittels zur Behandlung bakterieller Infektionen.

13. Die Verwendung nach Anspruch 12, worin die erwähnte N-acylierte Aminosäure wie in einem der Ansprüche 2 bis 8 definiert ist.

14. Die Verwendung nach Anspruch 12 oder Anspruch 13, worin das erwähnte Antibiotikum wie in einem dar Ansprüche 9 und 10 definiert ist.

15. Die Verwendung nach einem der Ansprüche 12 bis 14, worin das Gewichtsverhältnis zwischen der erwähnten N-acylierten Aminosäure und dem erwähnten Antibiotikum 0,1:1 bis 4:1 beträgt.
